# EUROPEAN PATENT APPLICATION

(11) **EP 0 735 040 A2**
(43) Date of publication of application: **02.10.1996**
(21) Application number: 96107948.0
(22) Date of filing: 10.12.1992
(51) Int. Cl.: C07F 9/6561, A61K 49/04, C07F 9/6524, A61K 49/00, A61K 47/48

(54) **Bicyclopolyazamacrocyclophosphonic acids, their complexes and conjugates, for use as contrast agents, and processes for their preparation**

(30) Priority: 10.12.1991 US 805551
(62) Divisional of application: 93901000.5
(71) Applicant: THE DOW CHEMICAL COMPANY, Midland, Michigan 48640 (US)
(72) Inventor: Kiefer, Garry E., Lake Jackson, TX 77566 (US); Garlich, Joseph R., Lake Jackson, TX 77566 (US); Simon, Jaime, Angleton, TX 77515 (US)
(74) Representative: Burford, Anthony Frederick

(57) **Abstract**

Bicyclopolyazamacrocyclophosphonic acid compounds are disclosed which may form inert complexes with Gd, Mn or Fe ions. The overall charge of the complex can be varied to alter the *in vivo* biolocalization. Such complexes can be covalently attached to an antibody, antibody fragment or other biologically active molecule to form conjugates. The complexes and conjugates are useful as contrast agents for diagnostic purposes. Processes for the preparation of the ligand, complex and conjugate are also disclosed.

## Description

This invention concerns ligands that are bicyclopolyazamacrocyclophosphonic acids, and complexes and conjugates thereof, for use as contrast agents in magnetic resonance imaging (MRI). Some ligands and complexes are also useful as oral care agents and as scale inhibiting agents in water treatment systems. To better understand this invention, a brief background on MRI is provided in the following section.

### Background

MRI is a non-invasive diagnostic technique which produces well resolved cross-sectional images of soft tissue within an animal body, preferably a human body. This technique is based upon the property of certain atomic nuclei (e.g. water protons) which possess a magnetic moment [as defined by mathematical equations; see G. M. Barrow, Physical Chemistry, 3rd Ed., McGraw-Hill, NY (1973)] to align in an applied magnetic field. Once aligned, this equilibrium state can be perturbed by applying an external radio frequency (RF) pulse which causes the protons to be tilted out of alignment with the magnetic field. When the RF pulse is terminated, the nuclei return to their equilibrium state and the time required for this to occur is known as the relaxation time. The relaxation time consists of two parameters known as spin-lattice (T1) and spin-spin (T2) relaxation and it is these relaxation measurements which give information on the degree of molecular organization and interaction of protons with the surrounding environment.

Since the water content of living tissue is substantial and variations in content and environment exist among tissue types, diagnostic images of biological organisms are obtained which reflect proton density and relaxation times. The greater the differences in relaxation times (T1 and T2) of protons present in tissue being examined, the greater will be the contrast in the obtained image [*J. Magnetic Resonance* 33, 83-106 (1979)].

It is known that paramagnetic chelates possessing a symmetric electronic ground state can dramatically affect the T1 and T2 relaxation rates of juxtaposed water protons and that the effectiveness of the chelate in this regard is related, in part, to the number of unpaired electrons producing the magnetic moment [*Magnetic Resonance Annual*, 231-266, Raven Press, NY (1985)]. It has also been shown that when a paramagnetic chelate of this type is administered to a living animal, its effect on the T1 and T2 of various tissues can be directly observed in the magnetic resonance (MR) images with increased contrast being observed in the areas of chelate localization. It has therefore been proposed that stable, non-toxic paramagnetic chelates be administered to animals in order to increase the diagnostic information obtained by MRI [*Frontiers of Biol. Energetics* l, 752-759 (1978); *J. Nucl. Med.* 25, 506-513 (1984); Proc. of NMR Imaging Symp. (Oct. 26-27, 1980); F. A. Cotton et al., *Adv. Inorg. Chem.* 634-639 (1966)]. Paramagnetic metal chelates used in this manner are referred to as contrast enhancement agents or contrast agents.

There are a number of paramagnetic metal ions which can be considered when undertaking the design of an MRI contrast agent. In practice, however, the most useful paramagnetic metal ions are gadolinium (Gd⁺³), iron (Fe⁺³), manganese (Mn⁺²) and (Mn⁺³), and chromium (Cr⁺³), because these ions exert the greatest effect on water protons by virtue of their large magnetic moments. In a non-complexed form (e.g. GdCl₃), these metal ions are toxic to an animal, thereby precluding their use in the simple salt form. Therefore, a fundamental role of the organic chelating agent (also referred to as a ligand) is to render the paramagnetic metal non-toxic to the animal while preserving its desirable influence on T1 and T2 relaxation rates of the surrounding water protons.

Art in the MRI field is quite extensive, such that the following summary, not intended to be exhaustive, is provided only as a review of this area and other compounds that are possibly similar in structure. US-A-4,899,755 discloses a method of alternating the proton NMR relaxation times in the liver or bile duct of an animal using Fe⁺³-ethylene-bis(2-hydroxyphenylglycine) complexes and its derivatives, and suggests among various other compounds the possible use of a pyridine macrocyclomethylenecarboxylic acid. US-A-4,880,008 (a CIP of US-A-4,899,755) discloses additional imaging data for liver tissue of rats, but without any additional complexes being shown. US-A-4,980,148 disclose gadolinium complexes for MRI which are non-cyclic compounds. C. J. Broan et al., *J. Chem. Soc., Chem. Commun.*, 1739-1741 (1990) describe some bifunctional macrocyclic phosphinic acid compounds. C. J. Broan et al., *J. Chem. Soc., Chem. Commun.*, 1738-1739 (1990) describe compounds that are triazabicyclo compounds. I. K. Adzamli et al., *J. Med. Chem.* 32, 139-144 (1989) describes acyclic phosphonate derivatives of gadolinium complexes for NMR imaging.

At the present time, the only commercial contrast agent available in the U.S.A. is the complex of gadolinium with diethylenetriaminepentaacetic acid (DTPA-Gd⁺³-MAGNEVIST™ by Schering AG). MAGNEVIST™ is considered as a non-specific/perfusion agent since it freely distributes in extracellular fluid followed by efficient elimination through the renal system. MAGNEVIST™ has proven to be extremely valuable in the diagnosis of brain lesions since the accompanying breakdown of the blood/brain barrier allows perfusion of the contrast agent into the affected regions. In addition to MAGNEVIST™, Guerbet is commercially marketing a macrocyclic perfusion agent (DOTAREM™) which presently is only available in Europe. A number of other potential contrast agents are in various stages of development.

Surprisingly, it has now been found that various bicyclopolyazamacrocyclophosphonic acid ligands can be contrast agents. Furthermore, these ligands may have their charge modified, i.e. by the structure of the ligand and metal selected, which can effect their ability to be more site specific. Specifically, the present invention is directed to novel ligands that are bicyclopolyazamacrocyclophosphonic acid compounds of the formula 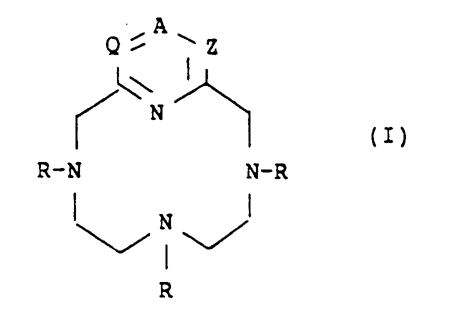 wherein: 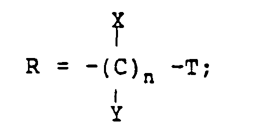 where:
X and Y are independently H, OH, C₁-C₃ alkyl or COOH;
n is an integer of 1, 2 or 3;
with the proviso that: when n is 2, then the sum of X and Y must equal two or more H; and
when n is 3, then the sum of X and Y must equal three or more H;
T is H, C₁-C₁₈ alkyl, COOH, OH, SO₃H, 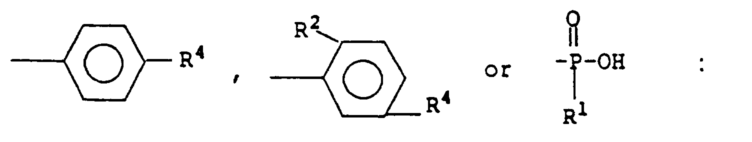
where:
R¹ is OH, C₁-C₅ alkyl or -O-(C₁-C₅ alkyl);
R⁴ is H, NO₂, NH₂, isothiocyanato, semicarbazido, thiosemicarbazido, maleimido, bromoacetamido or carboxyl;
R² is H or OH; with the proviso that when R² is OH, then the R term containing the R² must have all X and Y equal to H;
with the proviso that at least one T must be P(O)R¹OH, and with the proviso that when one T is 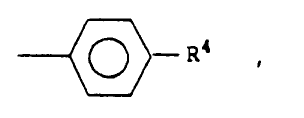 then one X or Y of that R term may be COOH and all other X and Y terms of that R term must be H;
A is CH, N, C-Br, C-Cl, C-OR³, C-OR⁸, N⁺-R⁵X⁻, 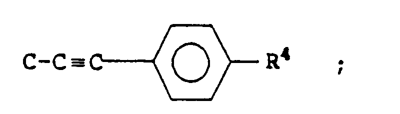
R³ is H, C₁-C₅ alkyl, benzyl, or benzyl substituted with at least one R⁴;
R⁴ is defined as above;
R⁵ is C₁-C₁₆ alkyl, benzyl, or benzyl substituted with at least one R⁴;
R⁸ is C₁-C₁₆ alkylamino;
X⁻ is Cl⁻, Br⁻, I⁻ or H₃CCO₂⁻;
Q and Z independently are CH, N, N⁺-R⁵X⁻, C-CH₂-OR³ or C-C(O)-R⁶;
R⁵ is defined as above;
R⁶ is -O-(C₁-C₃ alkyl), OH or NHR⁷;
R⁷ is C₁-C₅ alkyl or a biologically active material;
X⁻ is defined as above; or
pharmaceutically-acceptable salts thereof;
with the proviso that:
a) when Q, A or Z is N or N⁺-R⁵X⁻, then the other two groups must be CH;
b) when A is C-Br, C-Cl, C-OR³ or C-OR⁸, then both Q and Z must be CH;
c) the sum of the R⁴, R⁷ and R⁸ terms, when present, may not exceed one; and
d) only one of Q or Z can be C-C(O)-R⁶ and when one of Q or Z is C-C(O)-R⁶, then A must be CH.

When the above ligands of Formula (I) have at least two of the R terms T equal to PO₃H₂ [P(O)R¹OH where R¹ is OH] and the third T equal H, COOH or C₁-C₁₈ alkyl; A, Q and Z are CH; n is 1; and X and Y independently are H or C₁-C₃ alkyl; then the ligands are useful for oral care. Particularly preferred are those ligands where in the three R terms T is P(O)R¹OH, where R¹ is OH; n is 1; and X and Y are H. The use of these ligands is discussed and claimed in other copending applications.

When the above ligands of Formula (I) have:
in the R term at least two T equal P(O)R¹OH, where R¹ is OH, and in the other R term, T is COOH or P(O)R¹OH, and n, R¹, X, Y, A, Q and Z are defined as above;
in at least one R term T is P(O)R¹OH, where R¹ is OH, and in the other two R terms, T is COOH or P(O)R¹OH, and n, R¹, X, Y, A, Q and Z are defined as above; or
in the R term three T equal P(O)R¹OH, where R¹ is C₁-C₅ alkyl or -O-(C₁-C₅ alkyl), and n, R¹, X, Y, A, Q and Z are defined as above;
   then the ligands are useful as contrast agents.

Particularly preferred are those ligands of Formula (I) where:
X and Y are H;
n is 1; or
A, Q and Z are CH.

Bifunctional ligands of Formula (I) are desirable to prepare the conjugates of this invention. Such ligands must have:
one R term where the T moiety is 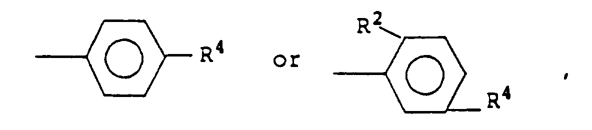 where R² and R⁴ are defined as above, especially where (a) in the two R terms not containing an R⁴ term, both T terms are P(O)R¹OH, where R¹ is defined as above and, preferably are the same moiety or (b) where in the two R terms not containing an R⁴ term, one T term is a COOH and the other T term is P(O)R¹OH, where R¹ is defined as above; preferably that moiety of the above T term containing R⁴ where one of X or Y of that term is COOH; and
also preferred are those ligands where n is 1 and/or the remaining X and Y terms are H; or
A is C-OR³ or C-OR⁸, where R³ and R⁸ are defined as above or 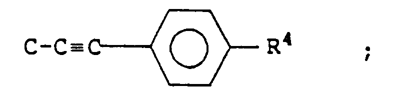 where R⁴ is defined as above; or
A is CH, and one of Q or Z is CH and the other is C-C(O)-R⁶, where R⁶ is defined as above;
especially those ligands where R⁶ is NHR⁷, where R⁷ is a biologically active material.

The ligands of Formula (I) may be complexed with various metal ions, such as gadolinium (Gd⁺³), iron (Fe⁺³), and manganese (Mn⁺²), with Gd⁺³ being preferred. The complexes so formed can be used by themselves or can be attached, by being covalently bonded to a larger molecule such as a dextran, a polypeptide or a biologically active molecule, including an antibody or fragment thereof, and used for diagnostic purposes. Such conjugates and complexes are useful as contrast agents.

The complexes and conjugates of this invention can be designed to provide a specific overall charge which advantageously influences the *in vivo* biolocalization and image contrast For example, when the metal ion is +3 the following can be obtained:
(A) an overall charge of -2 or more - when
   in three R terms T is P(O)R¹OH, where R¹ is OH, and n is 1; or
   in two R terms T is P(O)R¹OH, where R¹ is OH, in the third R term T is COOH, and n is 1; or
   in two R terms T is P(O)R¹OH, where R¹ is OH, in the third R term T is P(O)R¹OH, where R¹ is C₁-C₅ alkyl, and n is 1; or
   in two R terms T is P(O)R¹OH, where R¹ is OH, in the third R term T is P(O)R¹OH, where R¹ is -O-(C₁-C₅ alkyl), and n is 1; or
(B) an overall charge of -1 - when
   in one R term T is P(O)R¹OH, where R¹ is OH, and in the other two R terms T is P(O)R¹OH, where R¹ is -O-(C₁-C₅ alkyl), and n is 1; or
   in one R term T is P(O)R¹OH, where R¹ is OH, and in the other two R terms T is P(O)R¹OH, where R¹ is C₁-C₅ alkyl, and n is 1; or
   in one R term T is P(O)R¹OH, where R¹ is OH, and in the other two R terms T is COOH, and n is 1; or
(C) an overall neutral charge - when
   in the three R terms T is P(O)R¹OH, where R¹ is -O-(C₁-C₅ alkyl), and n is 1; or
   in the three R terms T is P(O)R¹OH, where R¹ is C₁-C₅ alkyl, and n is 1; or
(D) an overall charge of +1 - when
   one of A, Q or Z is N⁺-R⁵X⁻, where R⁵ and X⁻ are defined as above; and in one R term, the T moiety is P(O)R¹OH, where R¹ is C₁-C₅ alkyl or -O-(C₁-C₅ alkyl); and in the other two R terms, the T moiety is COOH or P(O)R¹OH, where R¹ is C₁-C₅ alkyl, -O-(C₁-C₅ alkyl); and all X and Y terms are H.

Both the complexes and conjugates may be formulated to be in a pharmaceutically acceptable form for administration to an animal.

Use of the ligands of Formula (I) with other metal ions for diagnosis of disease states such as cancer is possible. The use of those complexes and conjugates is discussed in another copending application.

The compounds of Formula (I) are numbered for nomenclature purposes as follows:

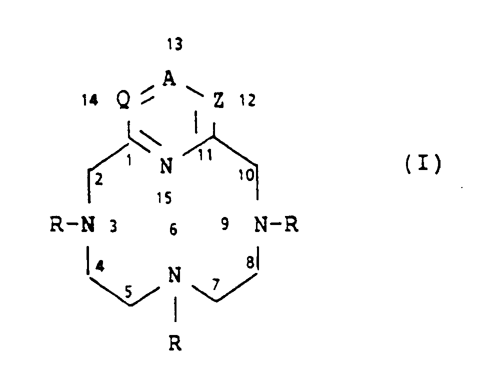

One aspect of the present invention concerns development of contrast agents having synthetic modifications to the paramagnetic chelate enabling site specific delivery of the contrast agent to a desired tissue. The advantage being increased contrast in the areas of interest based upon tissue affinity as opposed to contrast arising from non-specific perfusion which may or may not be apparent with an extracellular agent. The specificity of the ligand of Formula (I) may be controlled by adjusting the total charge and lipophilic character of the complex. The overall range of the charge of the complex is from -3 to +1. For example, for a complex having 2 or more PO₃H₂ groups, the overall charge is highly negative and bone uptake is expected; whereas when the overall charge of the complex is 0 (thus neutral), the complex may have the ability to cross the blood brain barrier and normal brain uptake may be possible.

Tissue specificity may also be realized by ionic or covalent attachment of the chelate to a naturally occurring or synthetic molecule having specificity for a desired target tissue. One possible application of this approach is through the use of chelate conjugated monoclonal antibodies which would transport the paramagnetic chelate to diseased tissue enabling visualization by MRI. In addition, attachment of a paramagnetic chelate to a macromolecule can further increase the contrast agent efficiency resulting in improved contrast relative to the unbound chelate. Recent work by Lauffer (US-A-4,880,008 and US-A-4,899,755) has demonstrated that variations in lipophilicity can result in tissue-specific agents and that increased lipophilic character favors non-covalent interactions with blood proteins resulting in enhancement of relaxivity.

Additionally, the present contrast agents of Formula (I) which are neutral in charge are particularly preferred for forming the conjugates of this invention since undesirable ionic interactions between the chelate and protein are minimized which preserves the antibody immunoreactivity. Also the present neutral complexes reduce the osmolarity relative to DTPA-Gd⁺³, which may alleviate the discomfort of injection.

While not wishing to be bound by theory, it is believed that when a charged complex of the invention is made (e.g. possibly -2 or -3 for bone, -1 for liver, or +1 for heart), the variations in that chelate ionic charge can influence biolocalization. Thus, if the antibody or other directing moiety is also specific for the same site, then the conjugate displays two portions to aid in site specific delivery.

The terms used in Formula (I) are further defined as follows. "C₁-C₃ alkyl", "C₁-C₅ alkyl", "C₁-C₁₈ alkyl", include both straight and branched chain alkyl groups. An "animal" includes a warmblooded mammal, preferably a human being.

"Biologically active material" refers to a dextran, peptide, or molecules that have specific affinity for a receptor, or preferably antibodies or antibody fragments.

"Antibody" refers to any polyclonal, monoclonal, chimeric antibody or heteroantibody, preferably a monoclonal antibody; "antibody fragment" includes Fab fragments and F(ab')₂ fragments, and any portion of an antibody having specificity toward a desired epitope or epitopes. When using the term "radioactive metal chelate/antibody conjugate" or "conjugate", the "antibody" is meant to include whole antibodies and/or antibody fragments, including semisynthetic or genetically engineered variants thereof. Possible antibodies are 1116-NS-19-9 (anti-colorectal carcinoma), 1116-NS-3d (anti-CEA), 703D4 (anti-human lung cancer), 704A1 (anti-human lung cancer), CC49 (anti-TAG-72), CC83 (anti-TAG-72) and B72.3. The hybridoma cell lines 1116-NS-19-9, 1116-NS-3d, 703D4, 704A1, CC49, CC83 and B72.3 are deposited with the American Type Culture Collection, having the accession numbers ATCC HB 8059, ATCC CRL 8019, ATCC HB 8301, ATCC HB 8302, ATCC HB 9459, ATCC HB 9453 and ATCC HB 8108, respectively.

As used herein, "complex" refers to a complex of the compound of Formula (I) complexed with a metal ion, where at least one metal atom is cheated or sequestered; "conjugate" refers to a metal ion chelate that is covalently attached to a biologically active agent, especially an antibody or antibody fragment. The terms "bifunctional coordinator", "bifunctional chelating agent" and "functionalized chelant" are used interchangeably and refer to compounds that have a chelant moiety capable of cheating a metal ion and a moiety covalently bonded to the chelant moiety that is capable of serving as a means to covalently attach to an antibody or antibody fragment.

The bifunctional chelating agents described herein (represented by Formula I) can be used to chelate or sequester the metal ions so as to form metal ion chelates (also referred to herein as "complexes"). The complexes, because of the presence of the functionalizing moiety (represented by R⁴ or R⁸ in Formula I), can be covalently attached to biologically active materials, such as dextran, molecules that have specific affinity for a receptor, or preferably covalently attached to antibodies or antibody fragments. Thus the complexes described herein may be covalently attached to an antibody or antibody fragment or have specific affinity for a receptor and are referred to herein as "conjugates".

As used herein, "pharmaceutically-acceptable salts" means any salt or mixtures of salts of a compound of formula (I) which is sufficiently non-toxic to be useful in therapy or diagnosis of animals, preferably mammals. Thus, the salts are useful in accordance with this invention. Representative of those salts formed by standard reactions from both organic and inorganic sources include, for example, sulfuric, hydrochloric, phosphoric, acetic, succinic, citric, lactic, maleic, fumaric, palmitic, cholic, palmoic, mucic, glutamic, gluconic acid, d-camphoric, glutaric, glycolic, phthalic, tartaric, formic, lauric, steric, salicylic, methanesulfonic, benzenesulfonic, sorbic, picric, benzoic, cinnamic acids and other suitable acids. Also included are salts formed by standard reactions from both organic and inorganic sources such as ammonium or 1-deoxy-1-(methylamino)-D-glucitol, alkali metal ions, alkaline earth metal ions, and other similar ions. Particularly preferred are the salts of the compounds of formula (I) where the salt is potassium, sodium, ammonium. Also included are mixtures of the above salts.

### Detailed Description of the Process

The compounds of Formula (I) are prepared by various processes. Typical general synthetic approaches to such processes are provided by the reaction schemes given below.

In Scheme 1, the compounds of Formula (I) are prepared wherein X and Y = H, n = 1 (but would also apply if n = 2 or 3 with the corresponding change in the reagent), T = PO₃H₂, and Q, A and Z = CH.

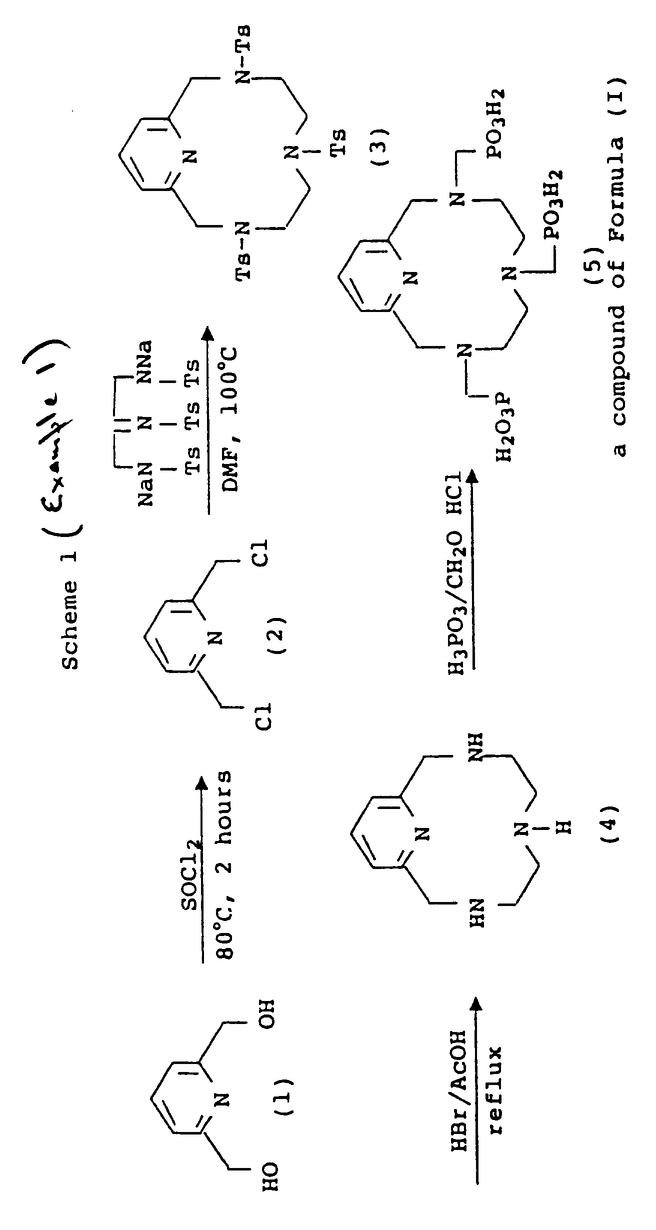

Scheme 2 prepares the compounds of Formula (I) wherein X and Y = H, n = 1 (but would also apply if n = 2 or 3 with the corresponding change in the reagent), T = 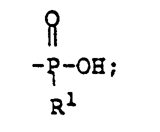 where R¹ = -O-(C₁-C₅ alkyl); and Q, A and Z = CH.

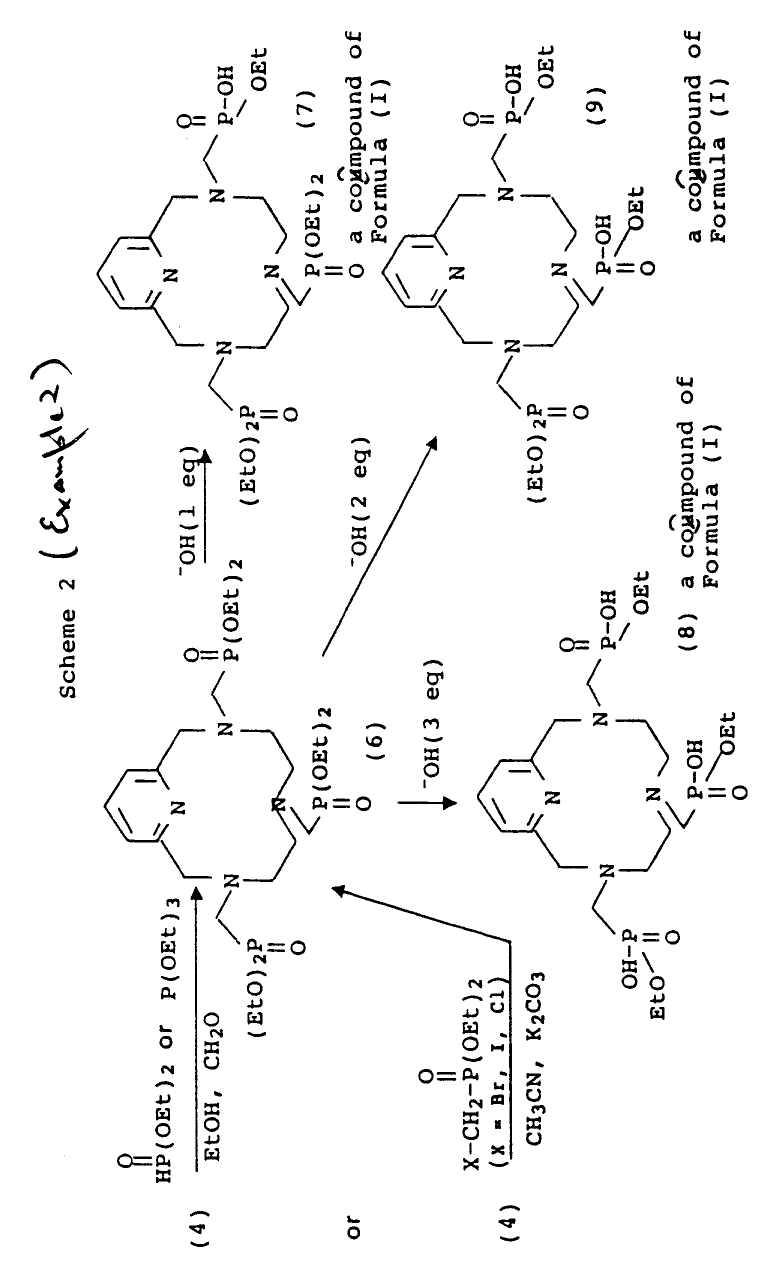

Scheme 3 prepares the compounds of Formula (I) wherein X and Y = H, n = 1 (but would also apply if n = 2 or 3 with the corresponding change in the reagent), T = 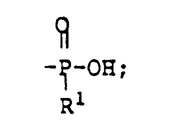 where R¹ = C₁-C₅ alkyl; and Q, A and Z = CH.

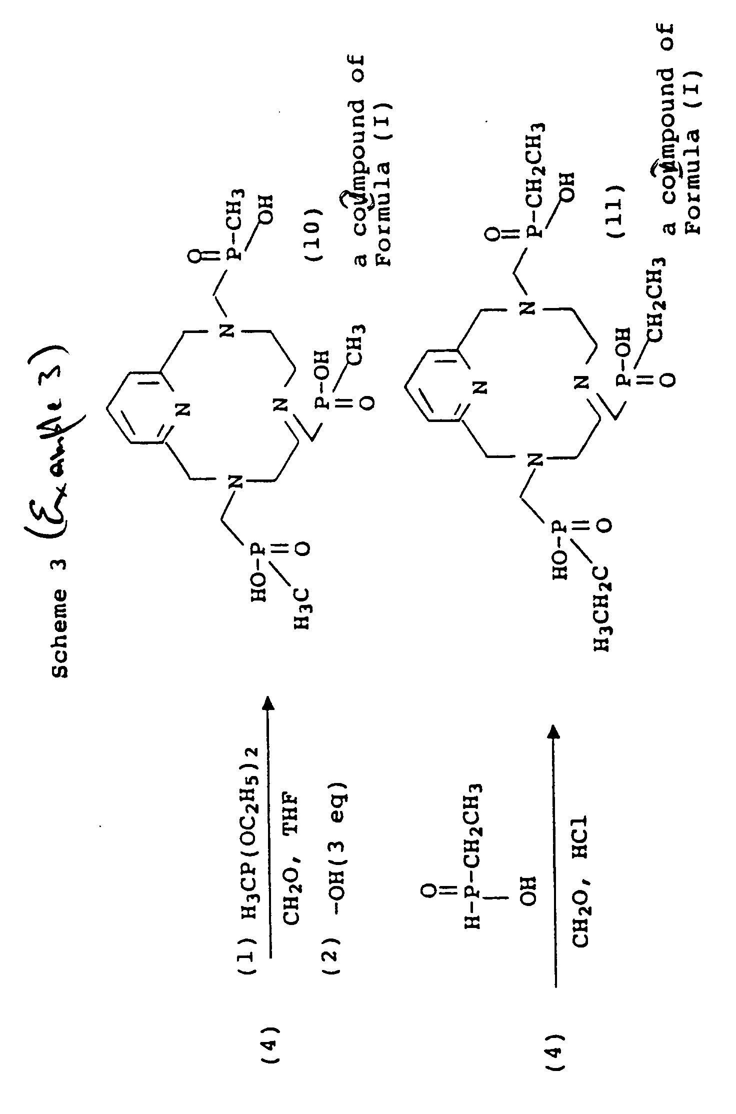

Scheme 4 prepares the compounds of Formula (I) wherein X and Y = H, n = 1 (but would also apply if n = 2 or 3 with the corresponding change in the reagent), T = 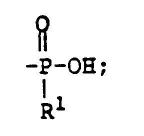 where R¹ = -O-(C₁-C₅ alkyl) or C₁-C₅ alkyl; A = C-Br, and Q and Z = CH.

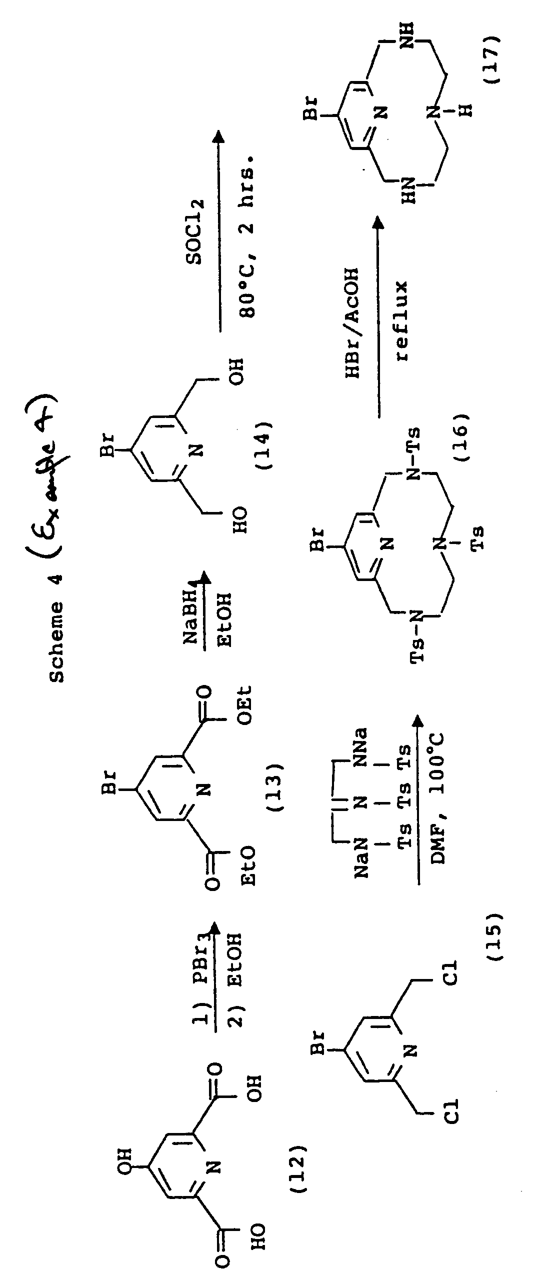

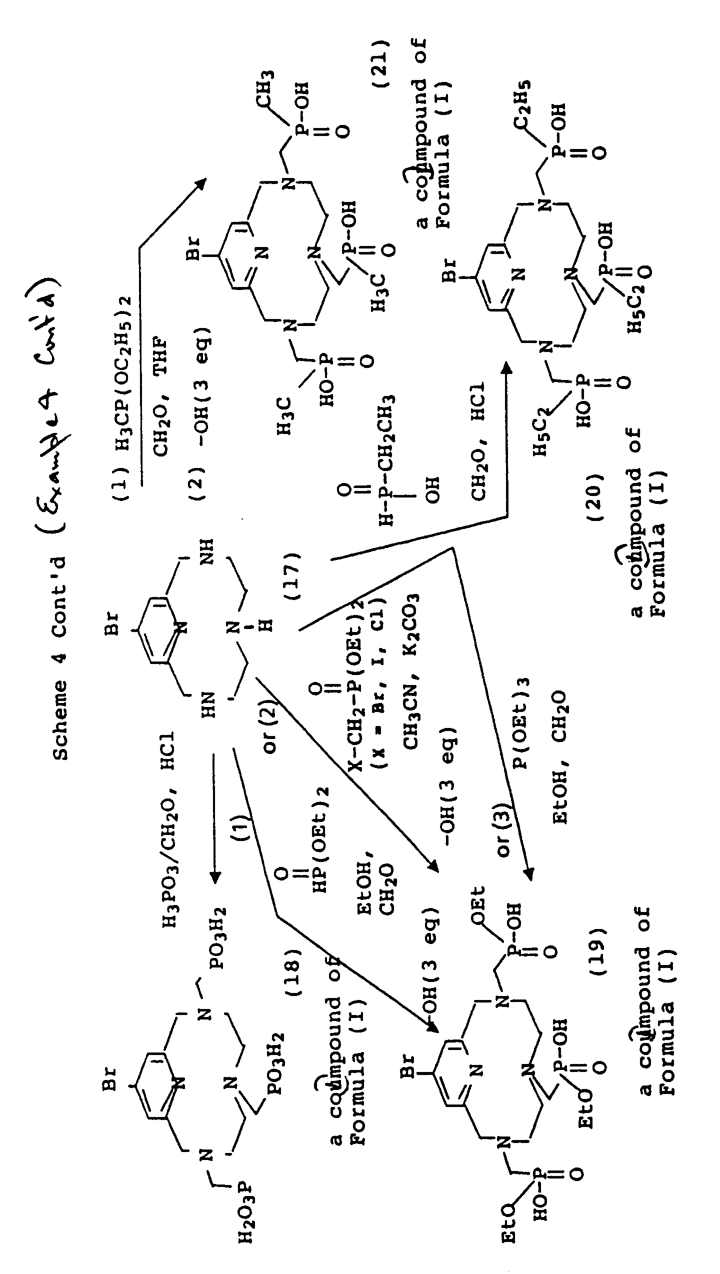

Scheme 5 prepares the compounds of Formula (I) wherein X and Y = H, n = 1 (but would also apply if n = 2 or 3 with the corresponding change in the reagent), T = 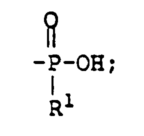 where R¹ = -O-(C₁-C₅ alkyl) or C₁-C₅ alkyl; A = 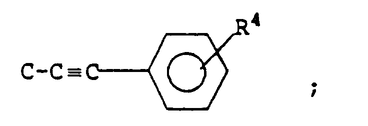 R⁴ = H, NO₂, NH₂ or SCN; and Q and Z = CH.

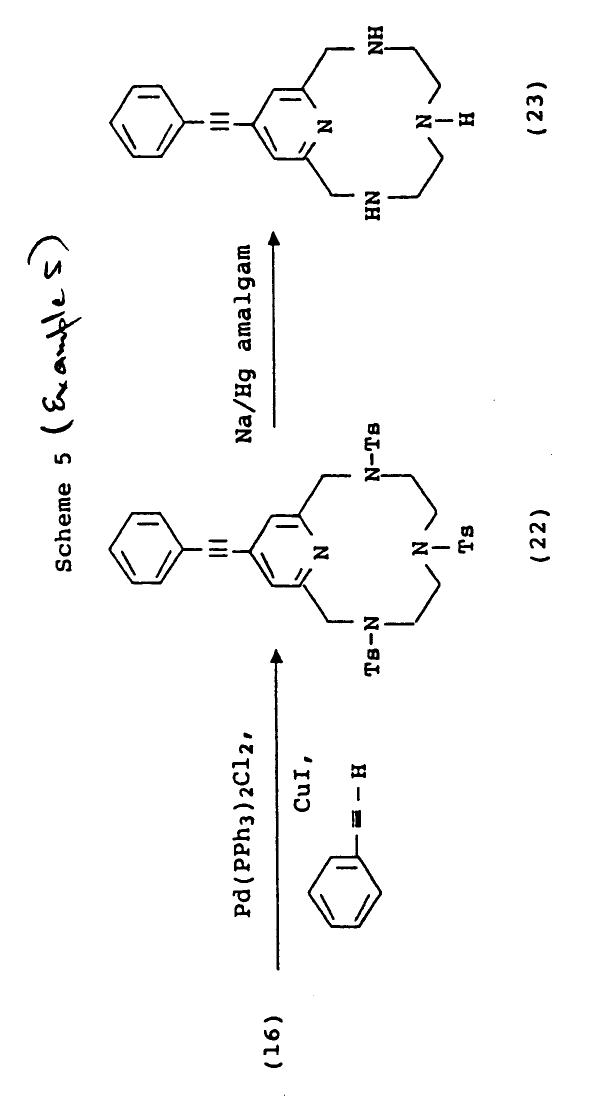

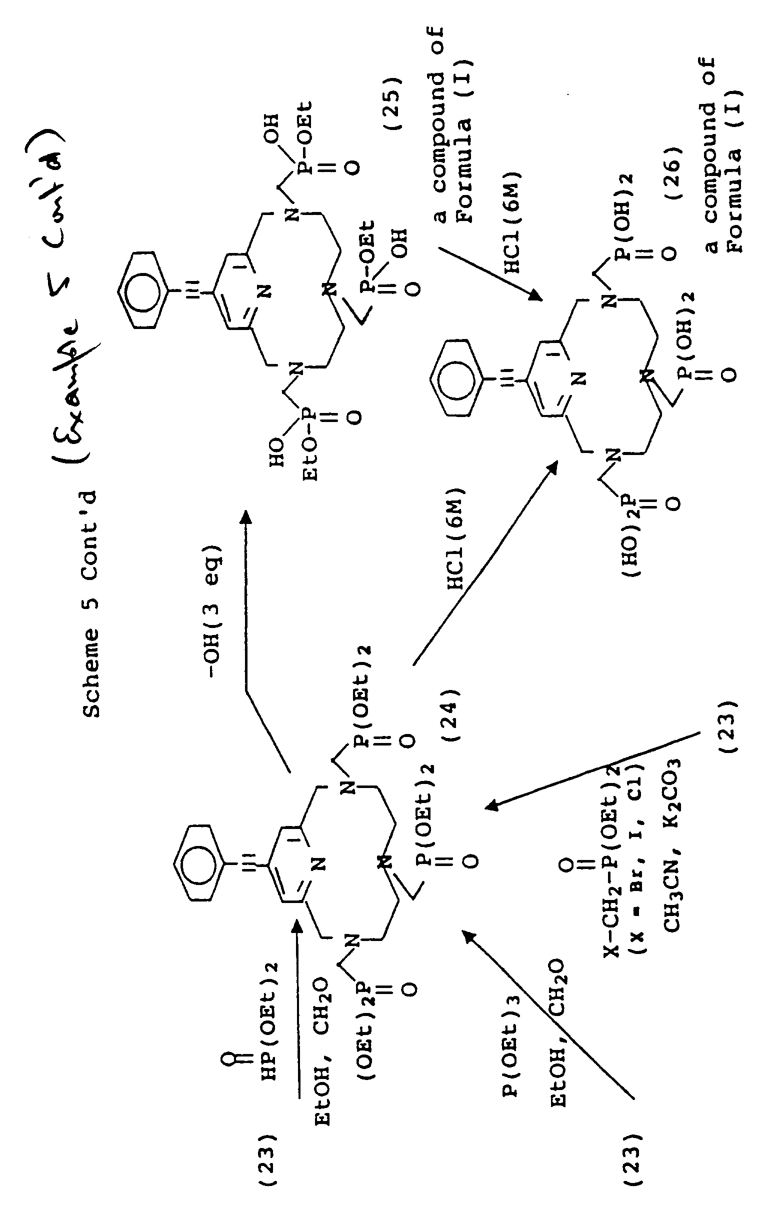

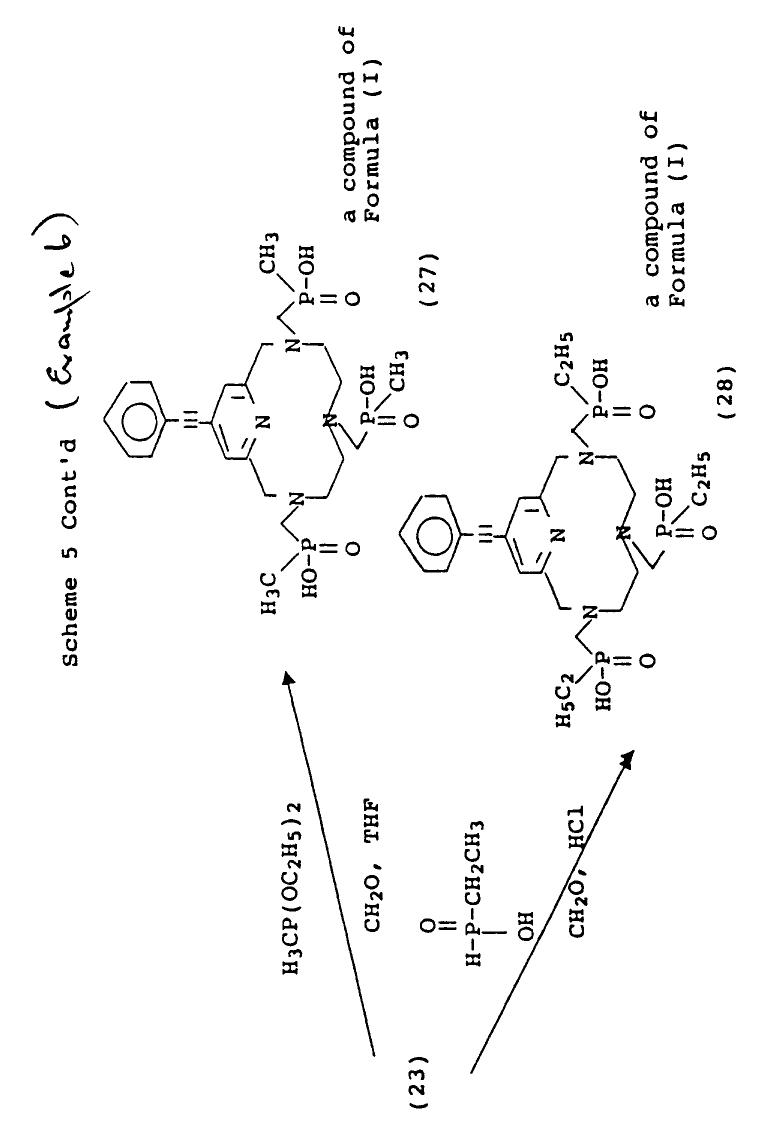

Scheme 6 prepares the compounds of Formula (I) wherein X and Y = H, n = 1 (but would also apply if n = 2 or 3 with the corresponding change in the reagent), T = 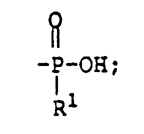 where R¹ = -O-(C₁-C₅ alkyl) or C₁-C₅ alkyl;
A = C-OR⁸, where R⁸ = C₁-C₅ alkylamino; and
Q and Z = CH.

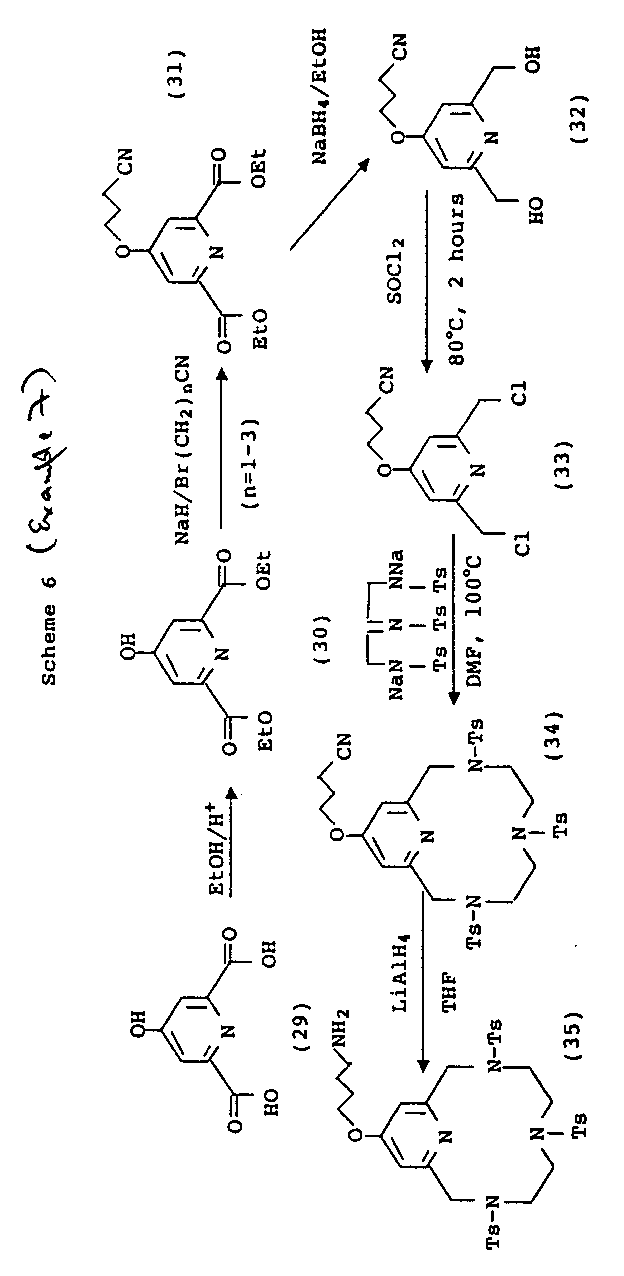

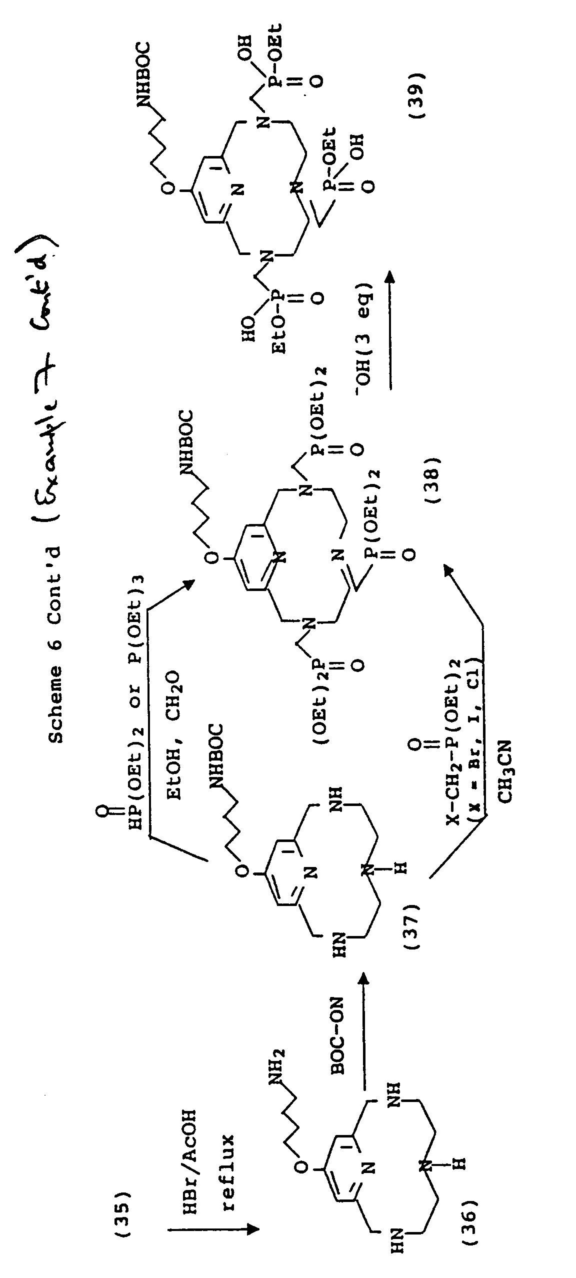

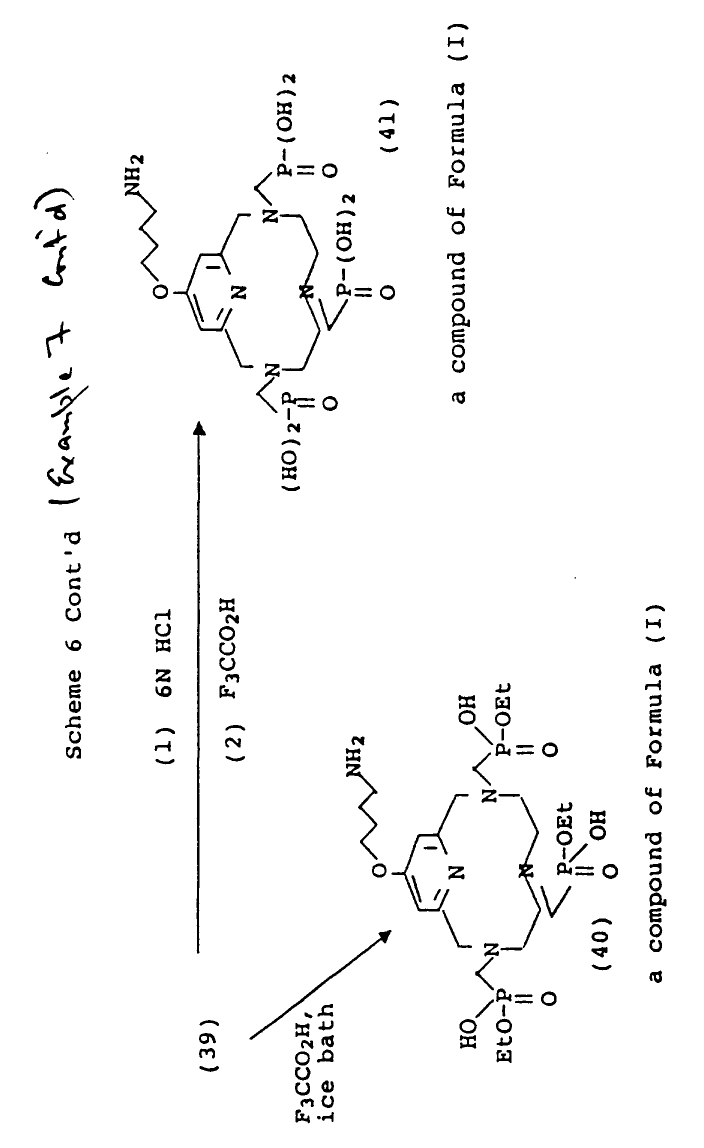

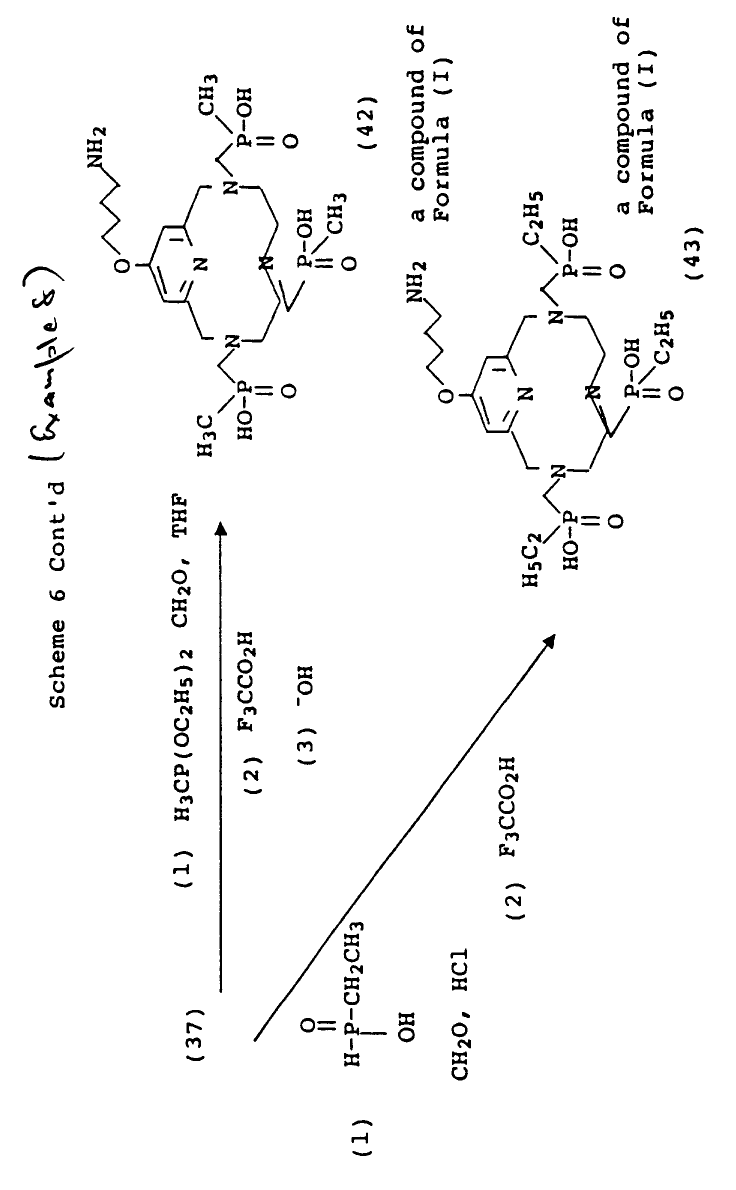

Scheme 7 prepares the compounds of Formula (I) wherein X and Y = H, n = 1 (but would also apply if n = 2 or 3 with the corresponding change in the reagent), T = 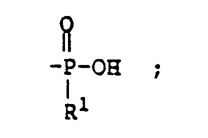 where R¹ = -OH, -O-(C₁-C₅ alkyl) or C₁-C₅ alkyl;
Z = C-C(O)-R⁶, where R⁶ = OH; and Q and A = CH.

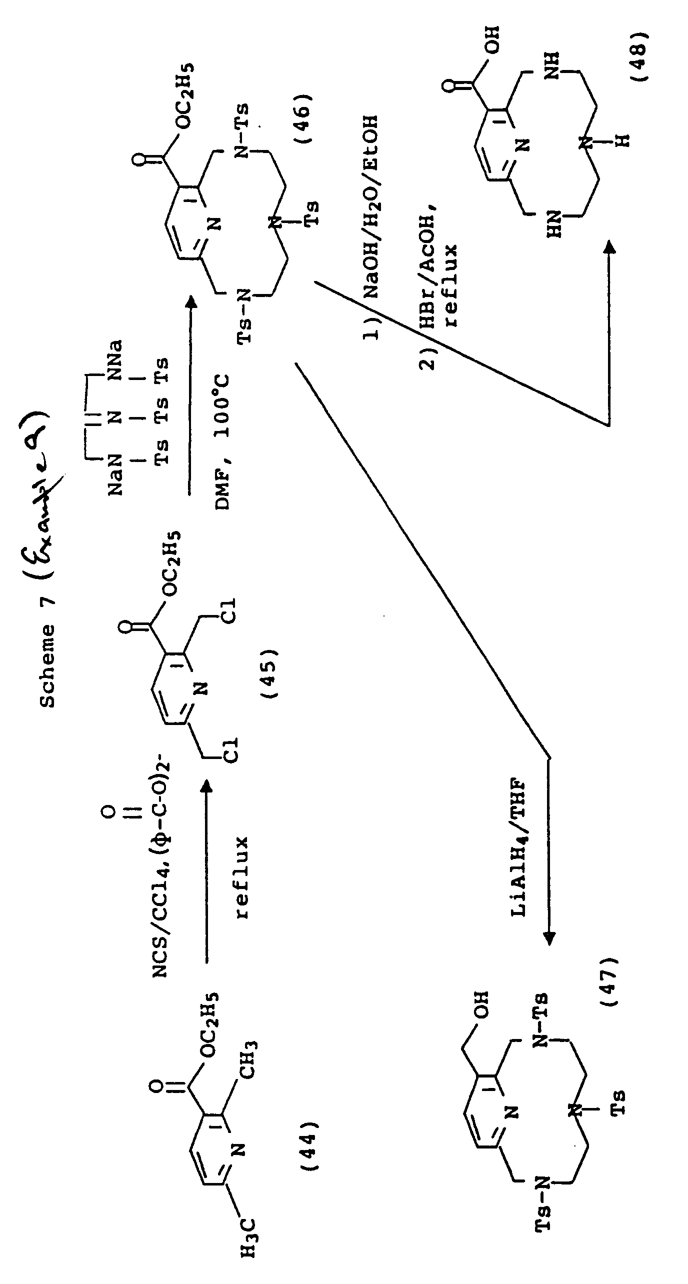

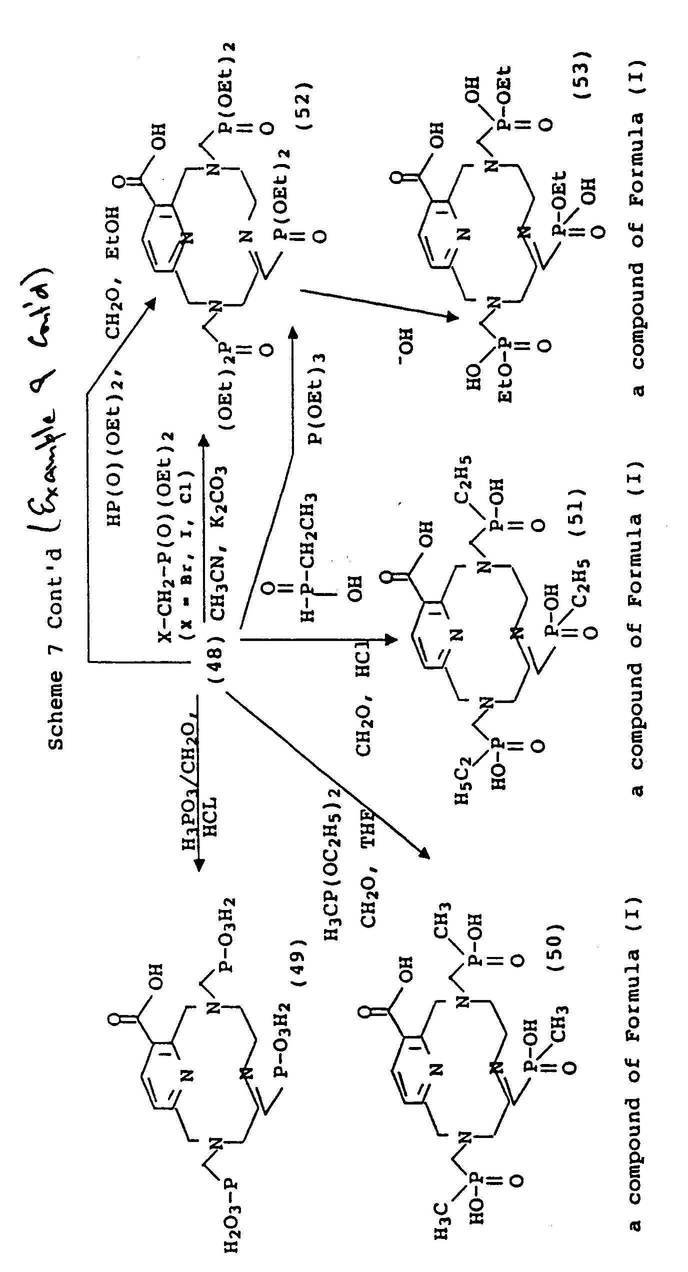

Scheme 8 prepares the compounds of Formula (I) wherein X and Y = H, n = 1 (but would also apply if n = 2 or 3 with the corresponding change in the reagent), T = 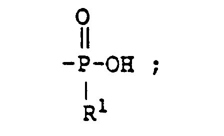 where R¹ = -OH, -O-(C₁-C₅ alkyl) or C₁-C₅ alkyl;
Z = C-CH₂-OR³ where R³ = benzyl; and
Q and A = CH.

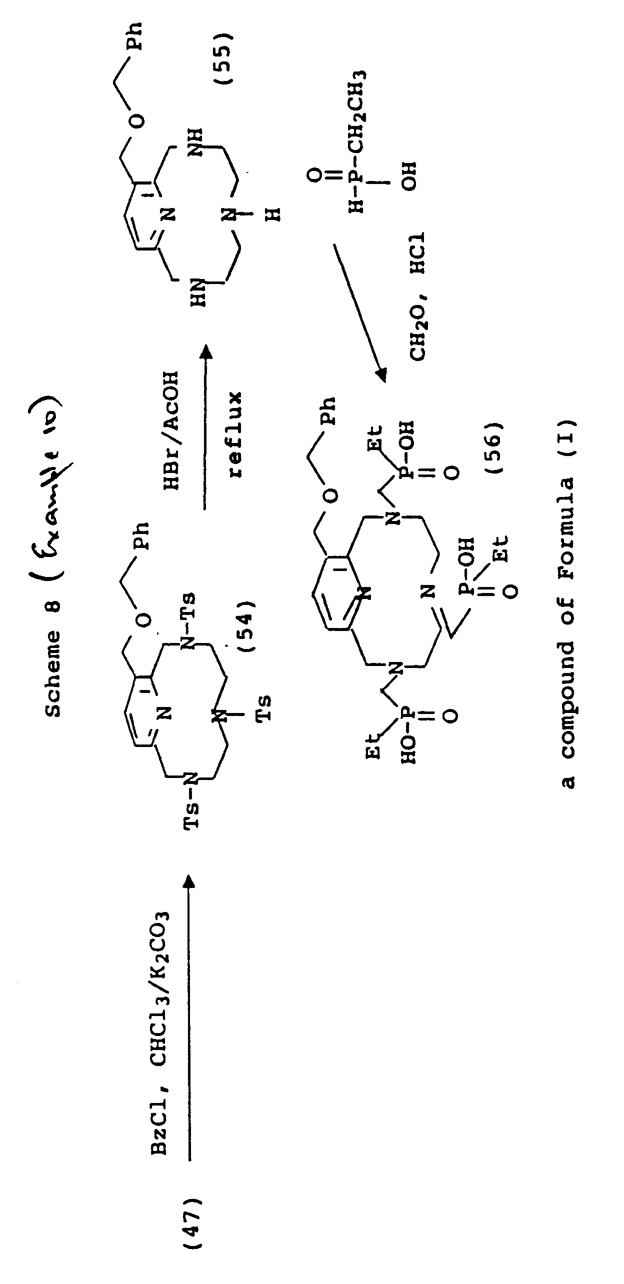

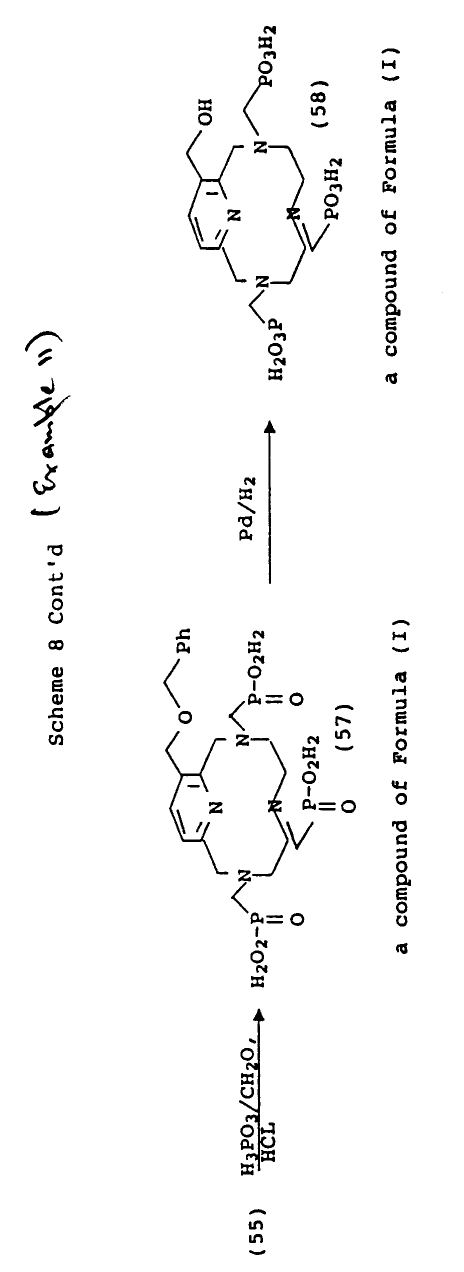

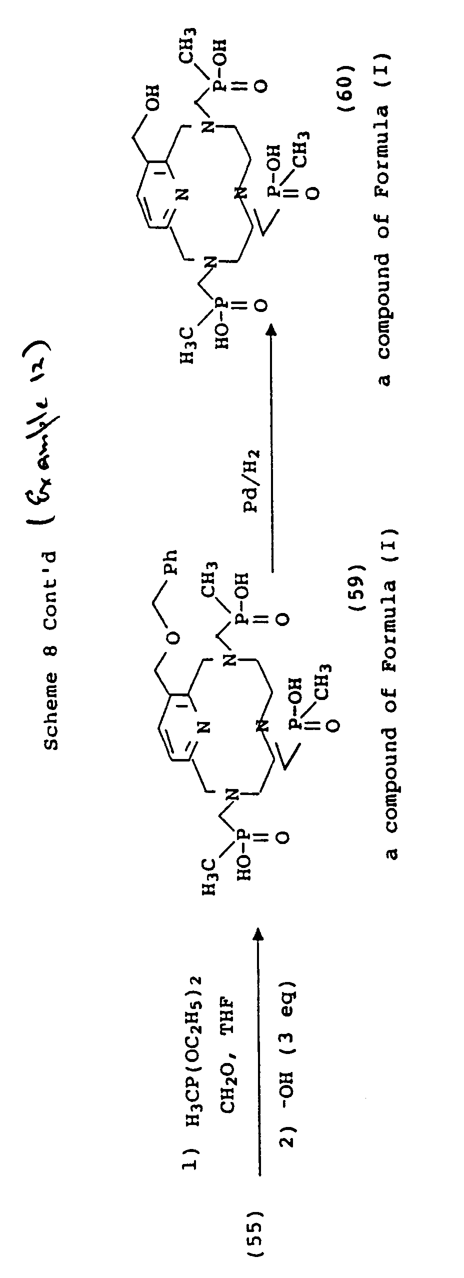

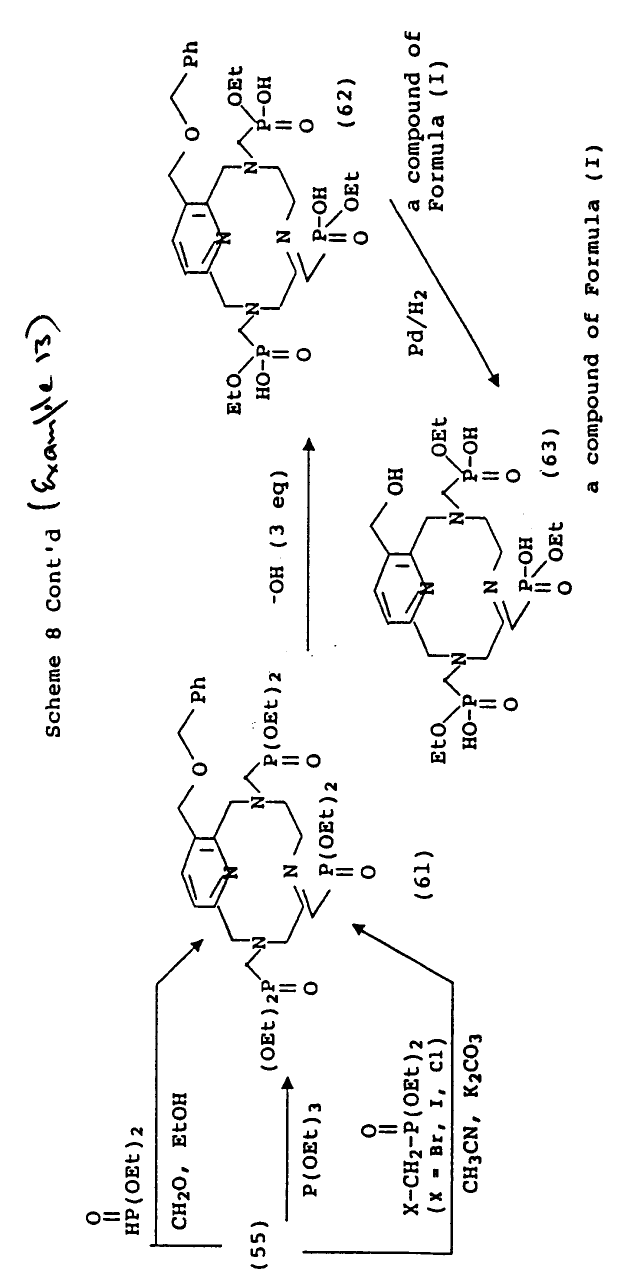

Scheme 9 prepares the compounds of Formula (I) wherein X and Y = H, n = 1 (but would also apply if n = 2 or 3 with the corresponding change in the reagent), T = 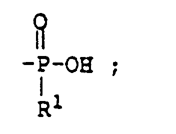 where R¹ = -OH, -O-(C₁-C₅ alkyl) or C₁-C₅ alkyl;
A = N or N-R⁵; R⁵ = C₁-C₁₆ alkyl halide; and
Q and Z = CH. 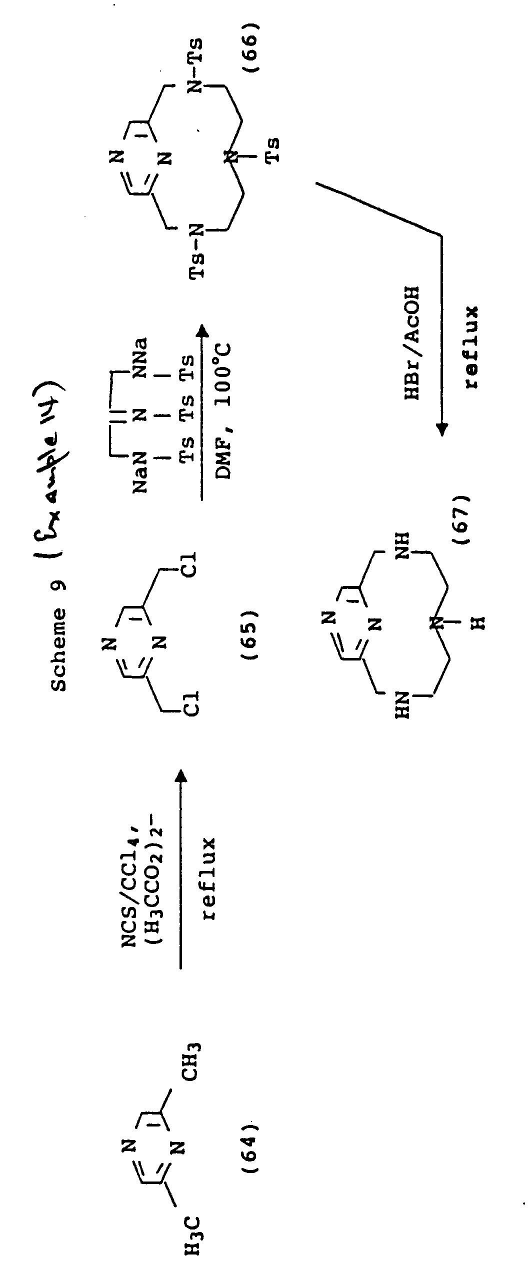 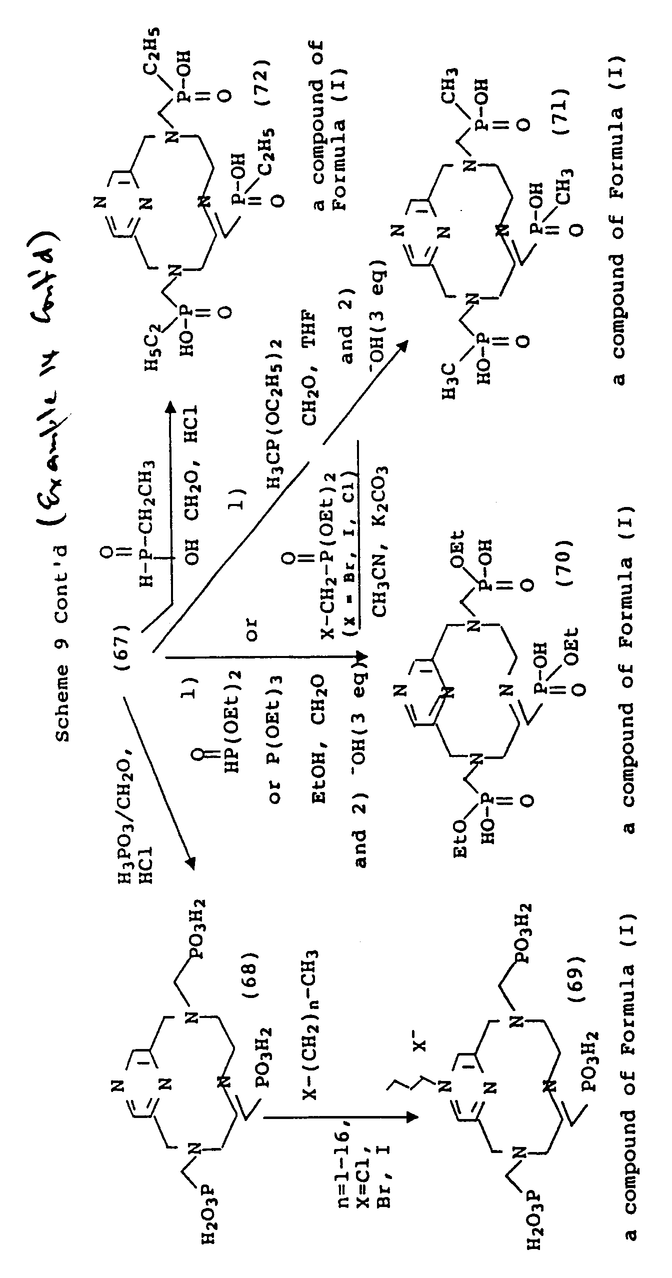 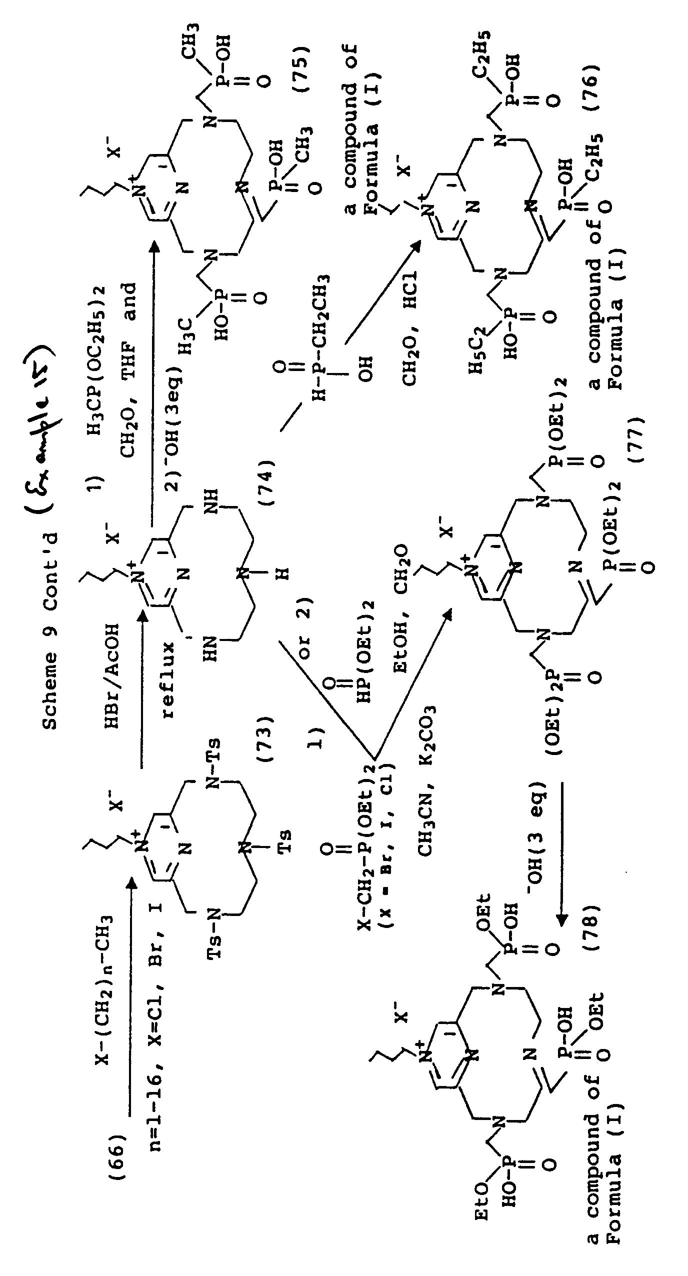 Scheme 10 prepares the compounds of Formula (I) wherein X and Y = H, n = 1 (but would also apply if n = 2 or 3 with the corresponding change in the reagent), T = 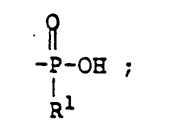 where R¹ = -OH, -O-(C₁-C₅ alkyl) or C₁-C₅ alkyl;
Q = N-R⁵; R⁵ = C₁-C₁₆ alkyl halide; and
A and Z = CH.

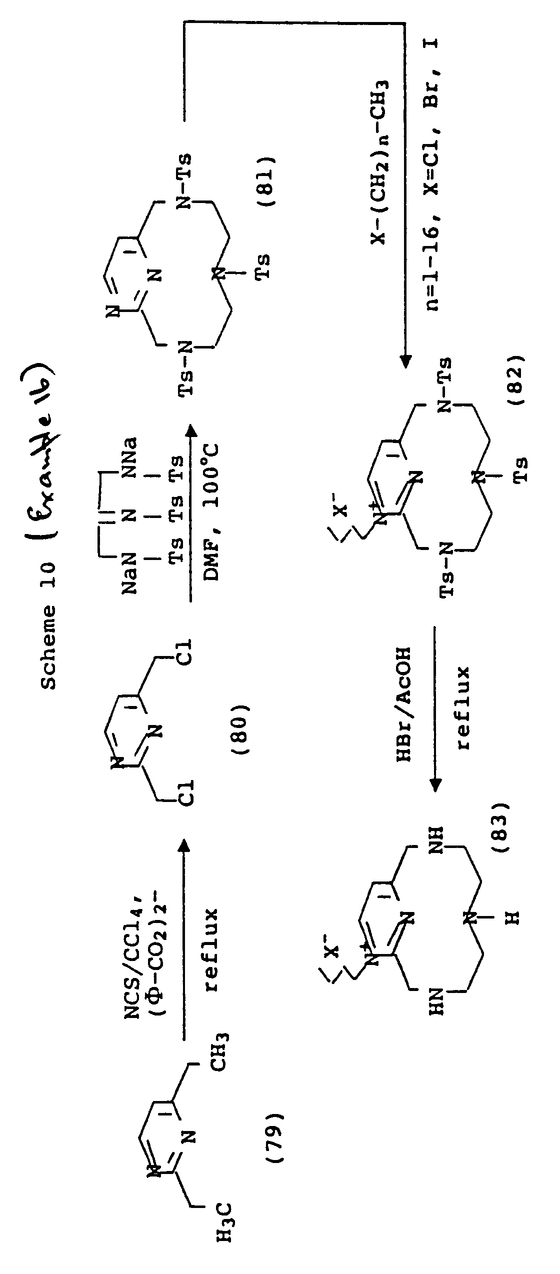

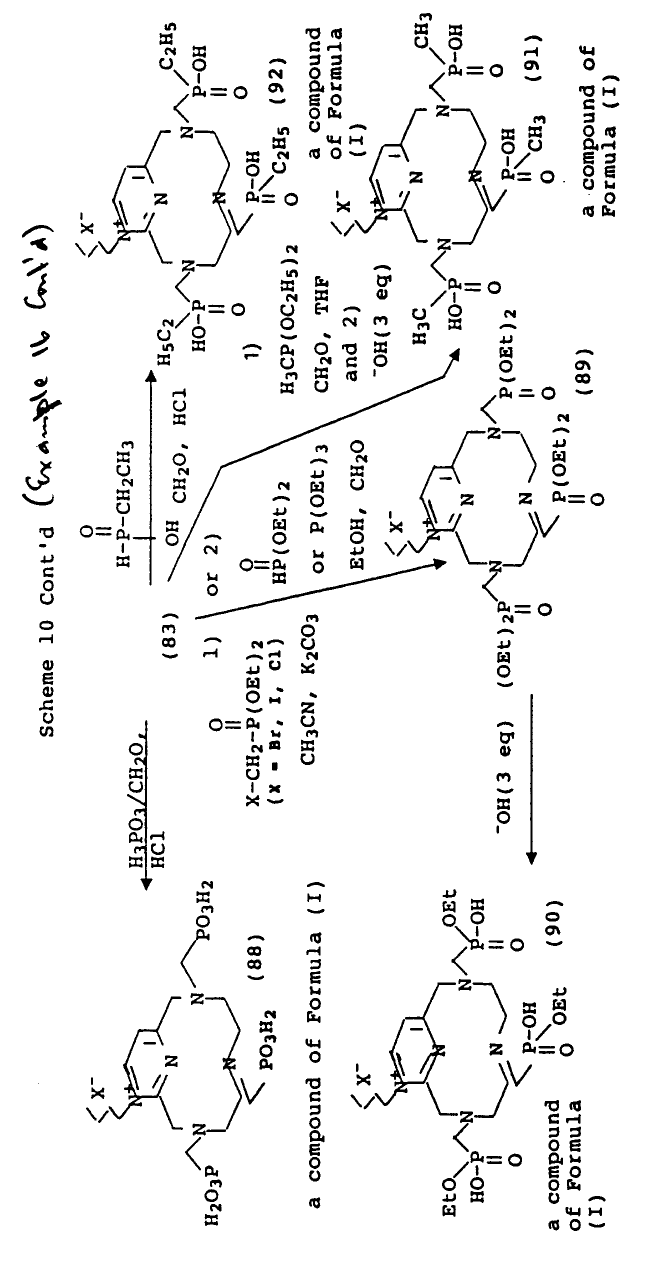

Scheme 11 prepares the compounds of Formula (I) wherein X and Y = H, n = 1 (but would also apply if n = 2 or 3 with the corresponding change in the reagent), T = 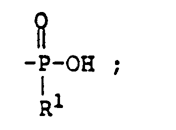 where R¹ = -OH, -O-(C₁-C₅ alkyl) or C₁-C₅ alkyl;
Q = N or N-R⁵, R⁵ = C₁-C₁₆ alkyl halide; and
A and Z = CH.

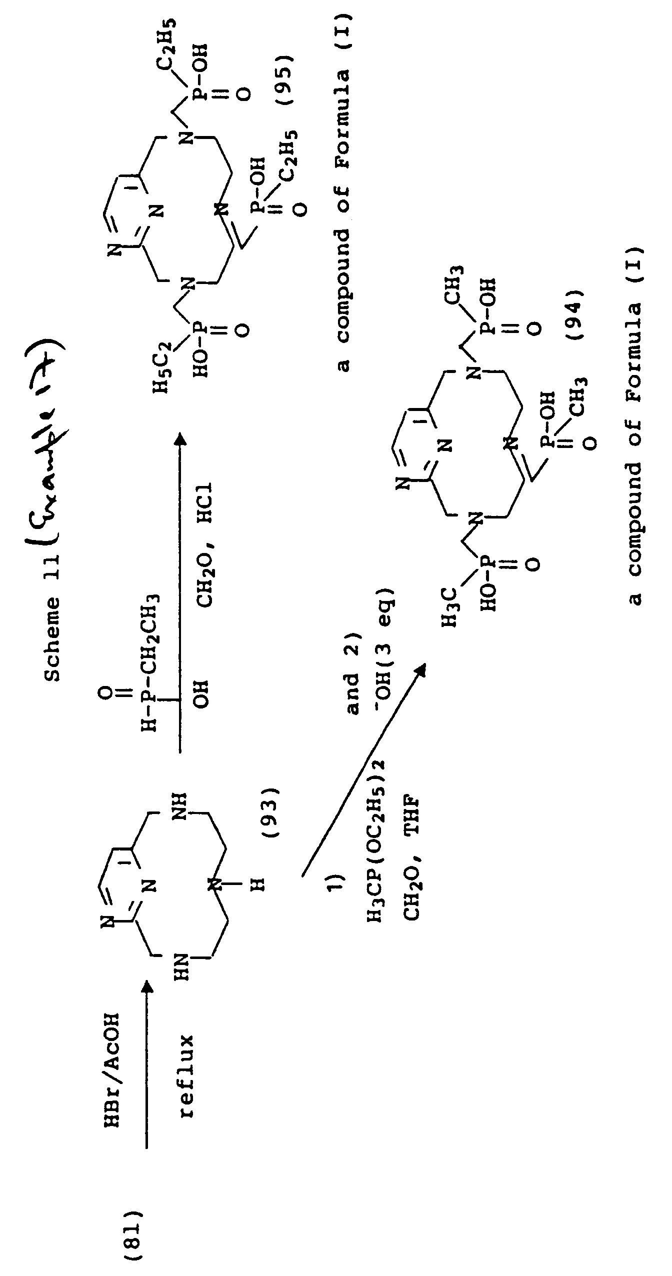

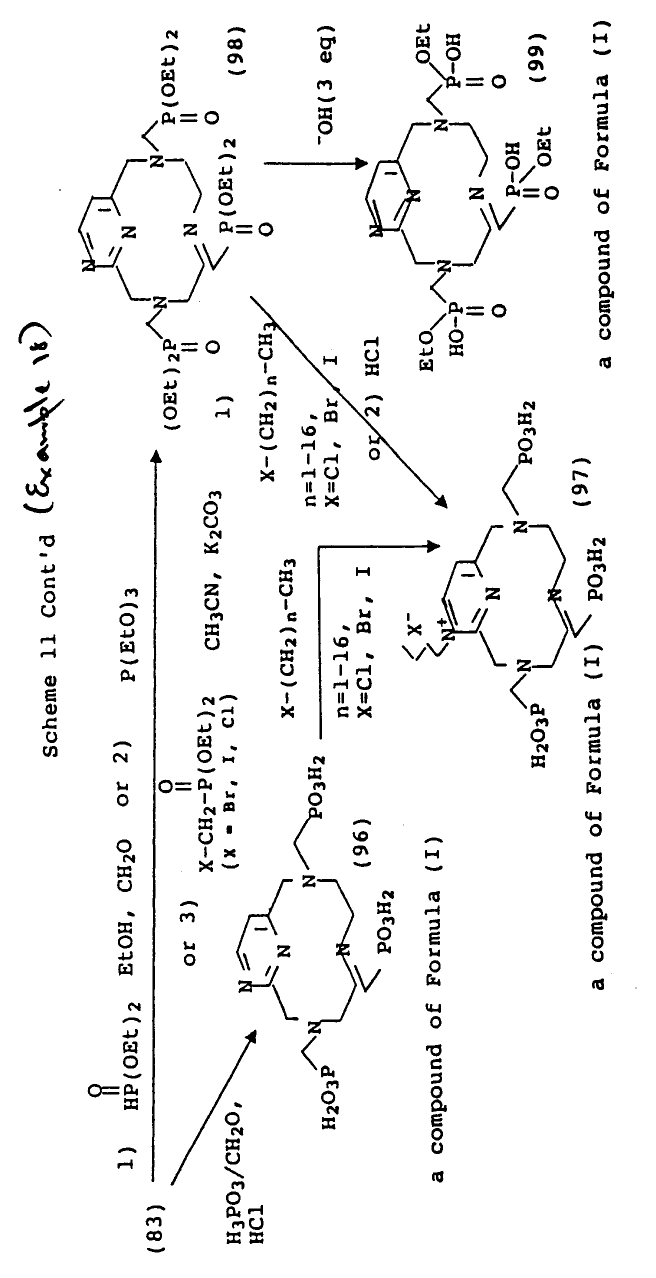

Scheme 12 prepares the compounds of Formula (I) wherein X and Y = H, n = 1 (but would also apply if n = 2 or 3 with the corresponding change in the reagent), R at the 3 position has T = 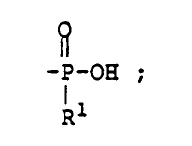 where R¹ = -OH or -O-(C₁-C₅ alkyl); and the other two R terms have T = COOH; and
A, Q and Z = CH.

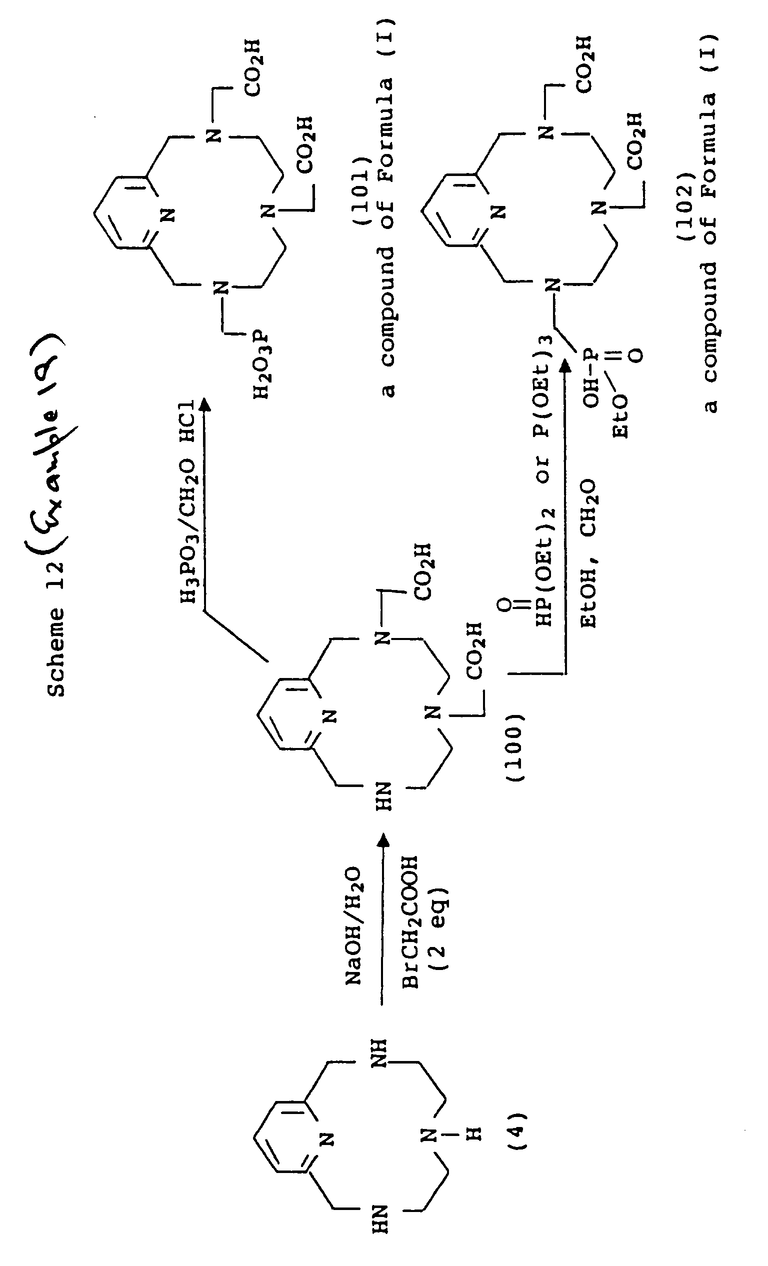

Scheme 13 prepares the compounds of Formula (I) wherein X and Y = H, n = 1 (but would also apply if n = 2 or 3 with the corresponding change in the reagent), R at the 3 and 6 positions have T = 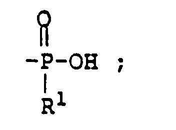 where R¹ = OH or -O-(C₁-C₅ alkyl); and the other R term at the 9 position has T = COOH; and
A, Q and Z = CH.

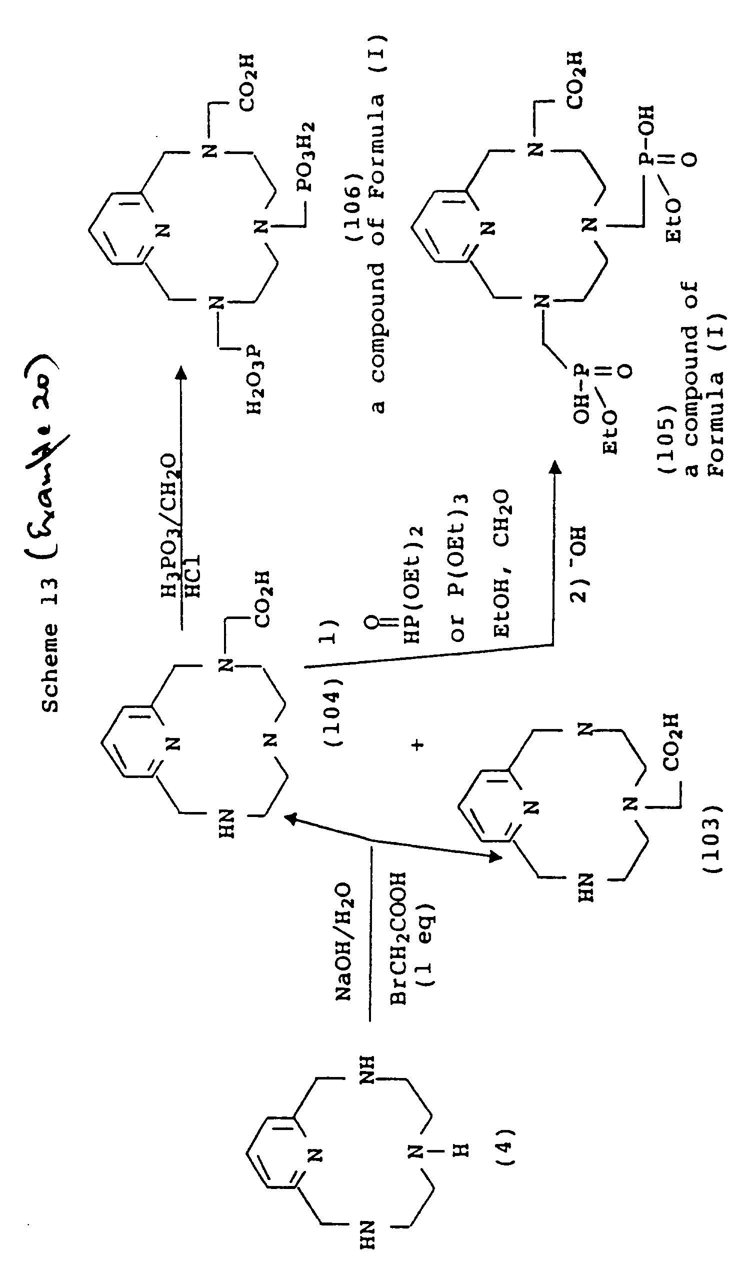

Scheme 14 prepares the compounds of Formula (I) wherein X and Y = H, n = 1 (but would also apply if n = 2 or 3 with the corresponding change in the reagent), R terms at the 3 and 9 positions have T = 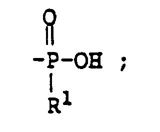 where R¹ = -OH or -O-(C₁-C₅ alkyl); and the other R term at the 6 position has T = COOH; and
A, Q and Z = CH.

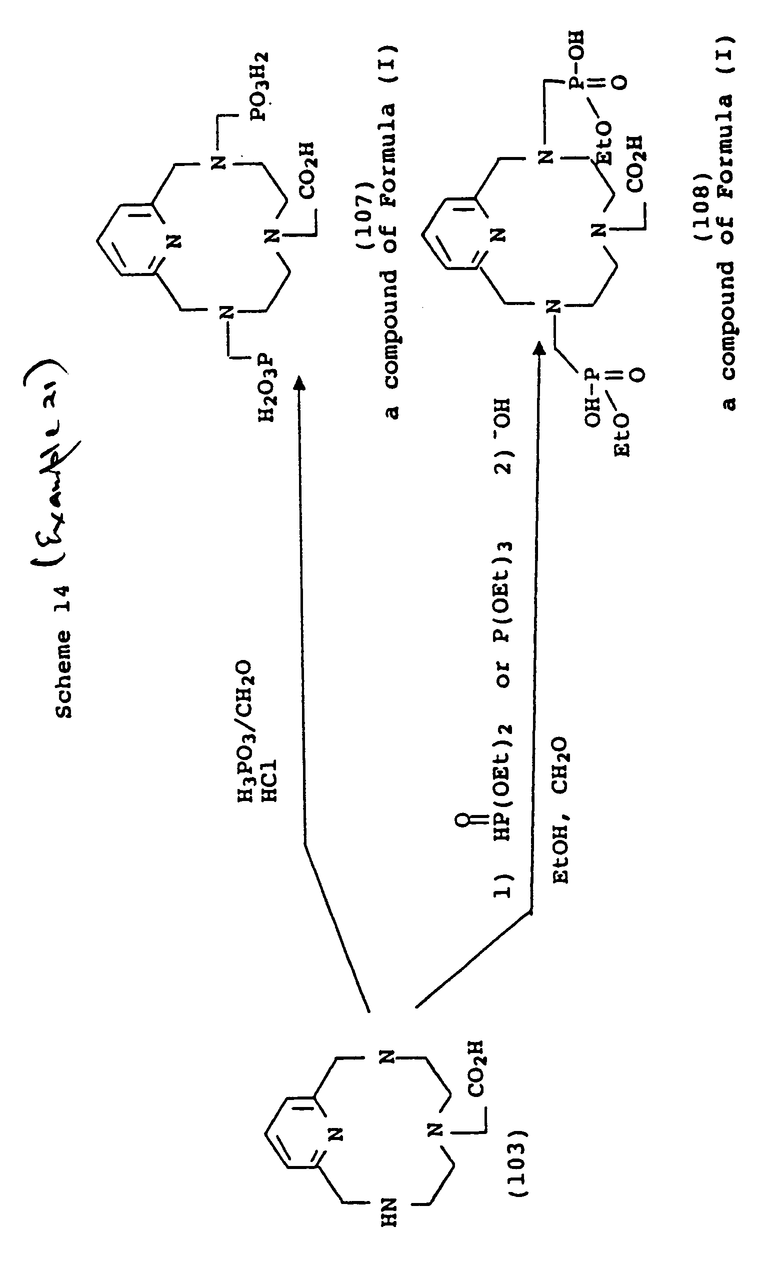

Scheme 15 prepares the compounds of Formula (I) wherein n = 1 (but would also apply if n = 2 or 3 with the corresponding change in the reagent), R terms at the 3 and 9 positions have T = 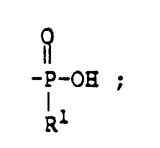 where R¹ = -OH or -O-(C₁-C₅ alkyl); and X and Y = H;
the R term at the 6 position has T = 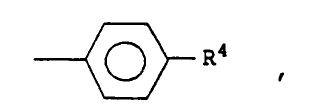 where R⁴ = NO₂ or NH₂; and one of X or Y = H and the other = COOH; and
A, Q and Z = CH.

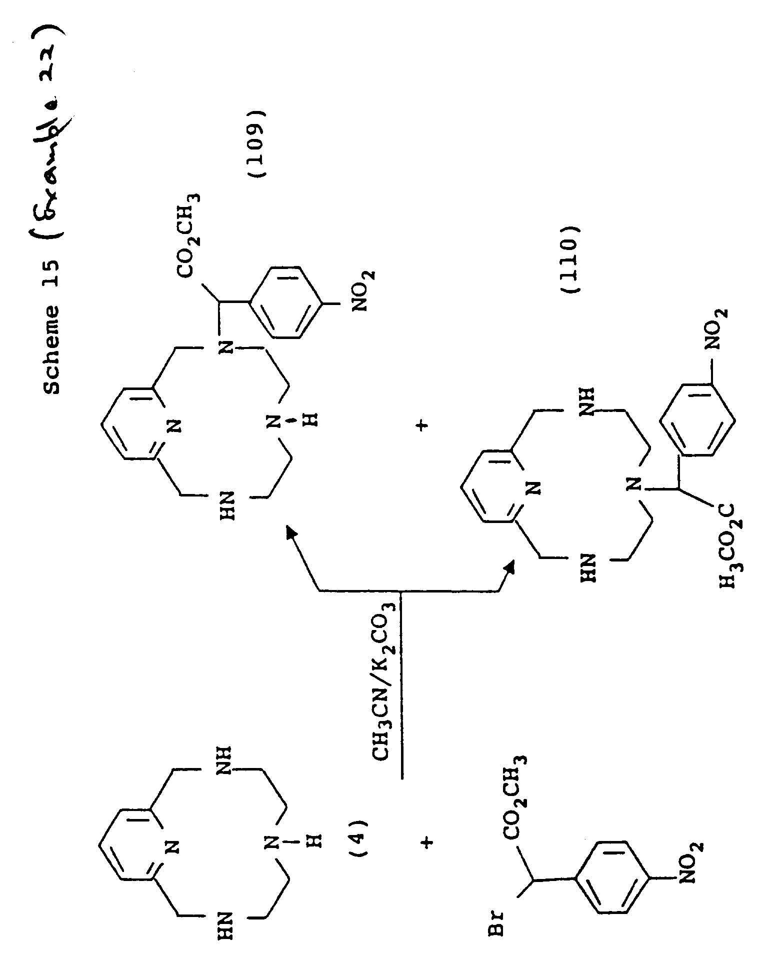

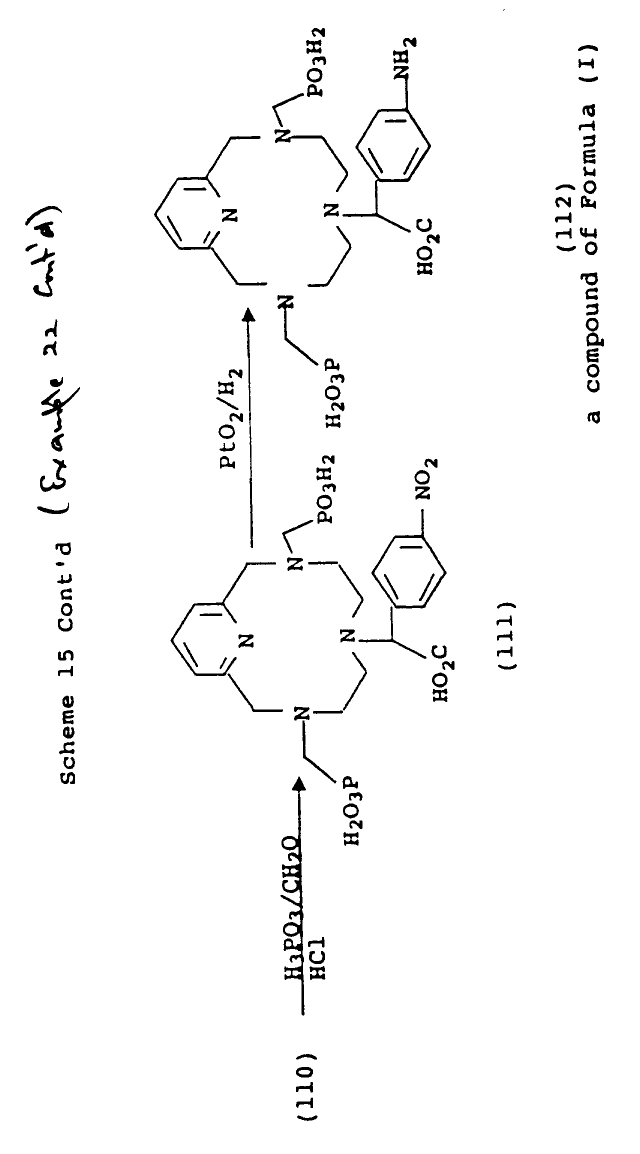

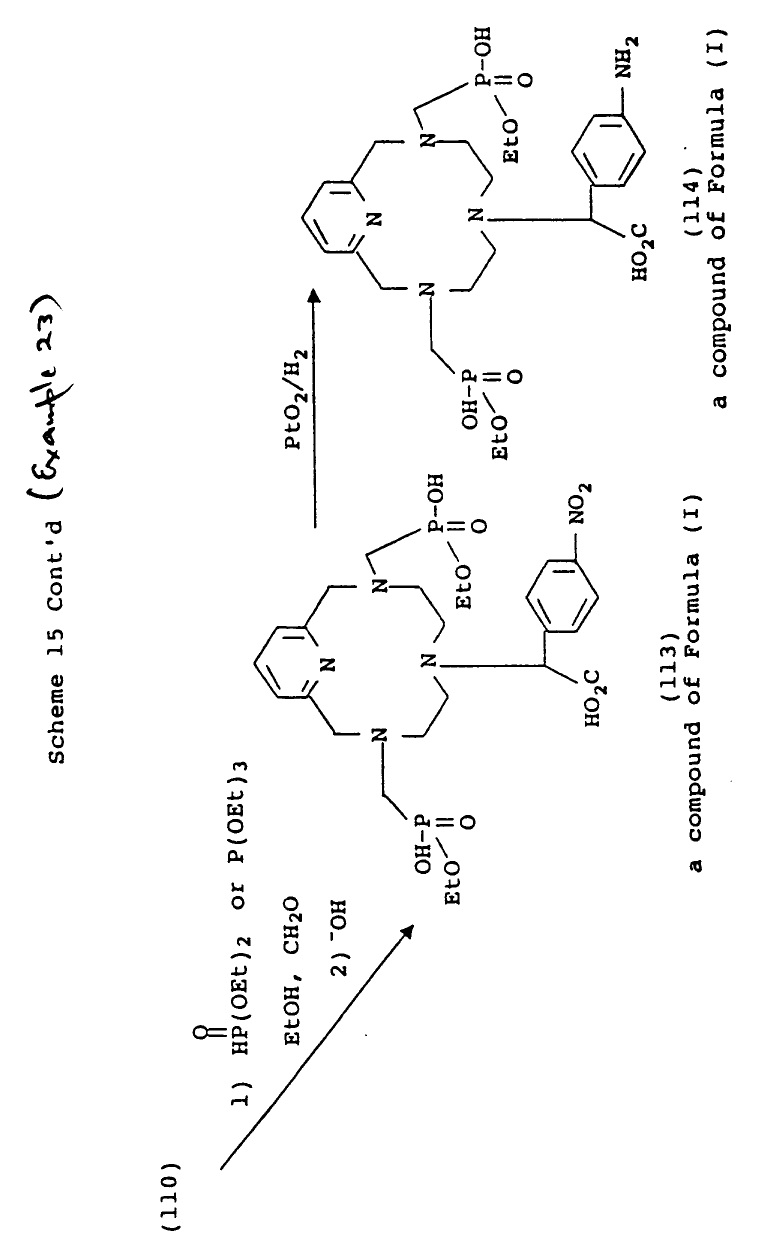

Scheme 16 prepares the compounds of Formula (I) wherein n = 1 (but would also apply if n = 2 or 3 with the corresponding change in the reagent), R terms at the 3 and 6 positions have T = 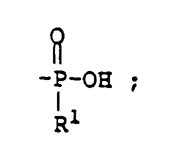 where R¹ = -OH or -O-(C₁-C₅ alkyl); and X and Y = H;
the R term at the 9 position has T = 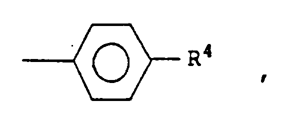 where R⁴ = NO₂ or NH₂; and one of X or Y = H and the other = COOH,
A, Q and Z = CH.

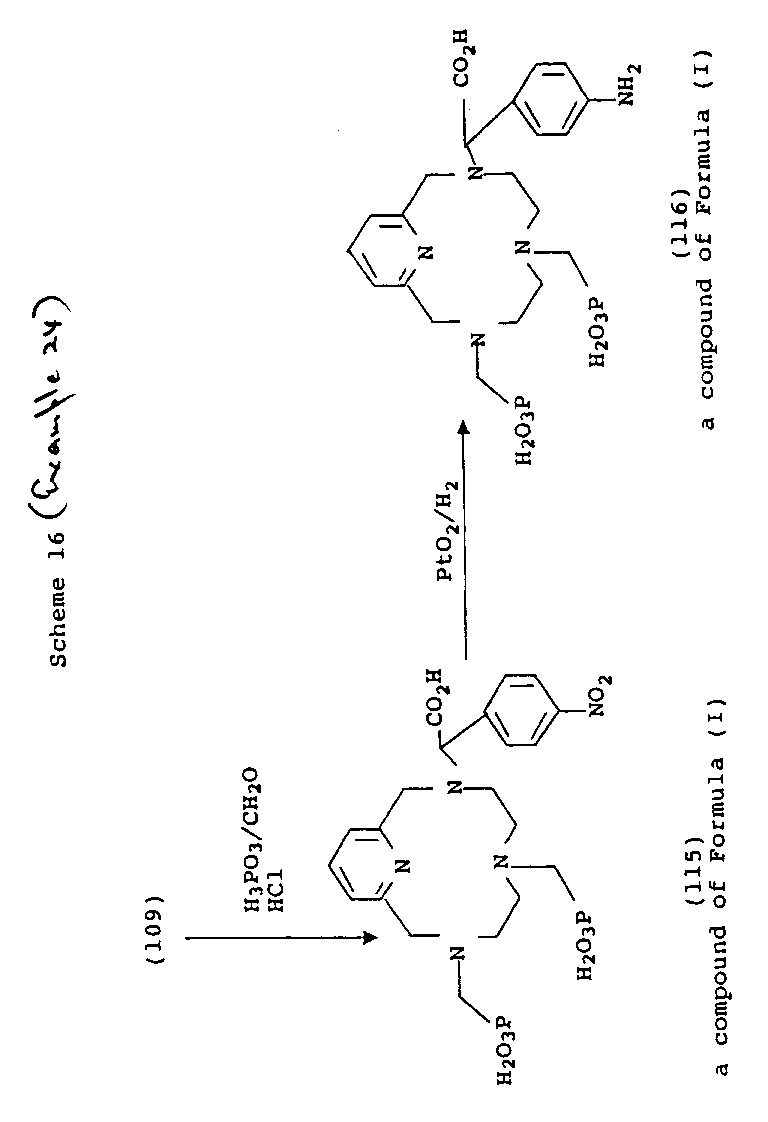

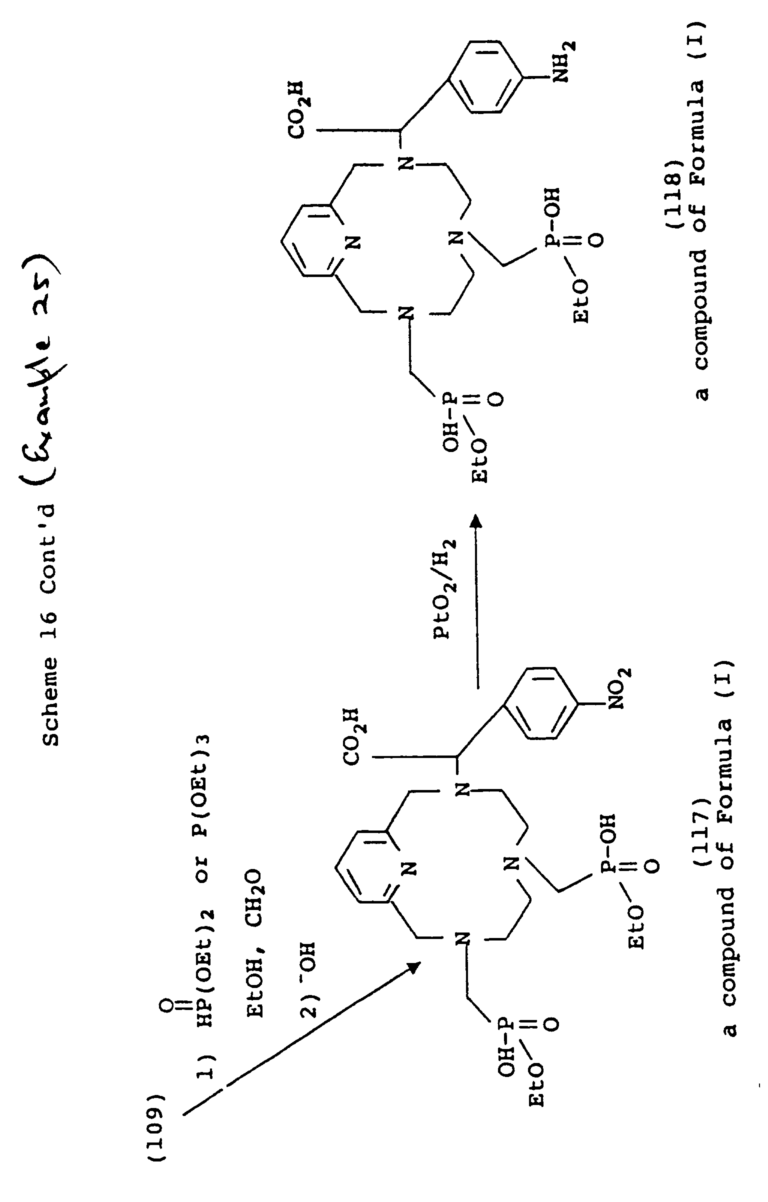

Scheme 17 prepares the compounds of Formula (I) wherein n = 1 (but would also apply if n = 2 or 3 with the corresponding change in the reagent), the R term at the 6 position has T = 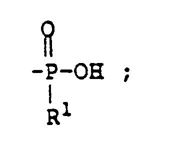 where R¹ = -OH; and X and Y = H;
the R term at the 3 and 9 positions have T = COOH; and
A, Q and Z = CH.

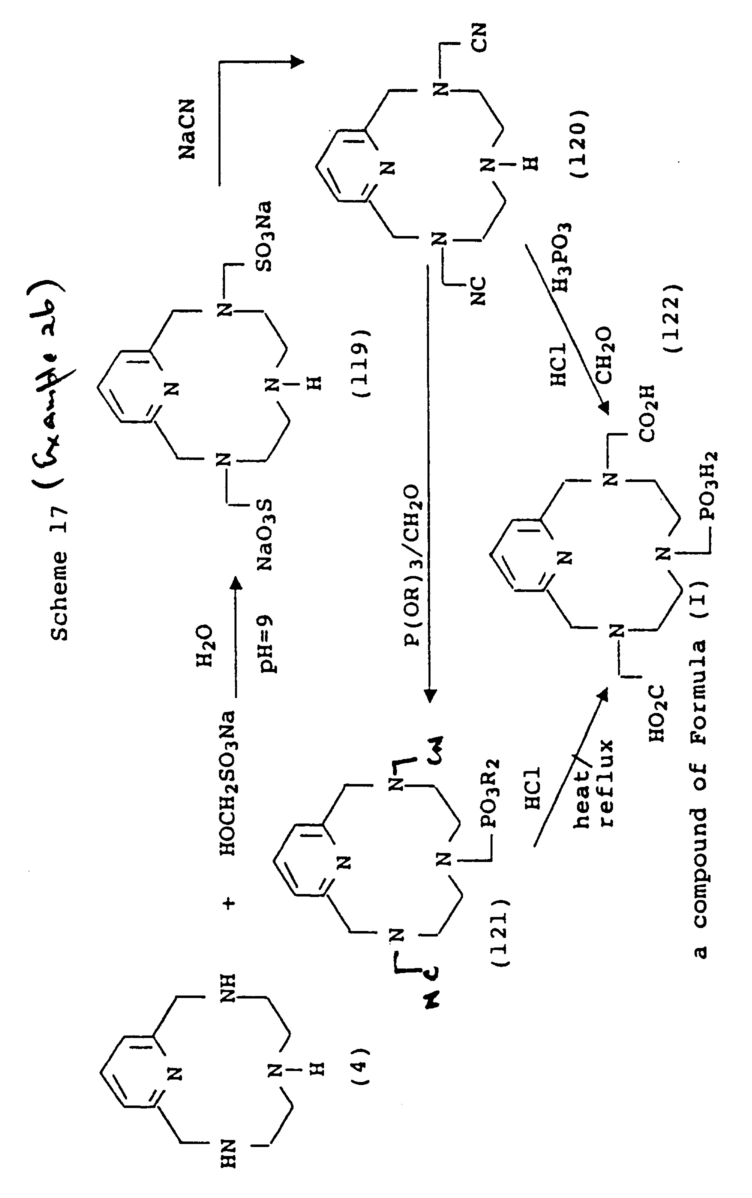

In the above Schemes, the general process description illustrates specific steps that may be used to accomplish a desired reaction step. The general description of these process steps follows.

The synthetic Scheme 1 begins with a halogenation of commercially available bis-pyridyl alcohol (1) using thionyl chloride. Similar procedures for converting an alcohol to an electrophilic substrate, such as treatment with toluenesulfonyl chloride, HBr or HCl, should also result in a similarly reactive product which would work well in subsequent ring closure reactions. Macrocyclization procedures are numerous in the literature and the desired tetraazamacrocycle (3) was prepared according to the method of Stetter et al., *Tetrahedron* 37, 767-772 (1981). More general procedures have since been published which give good yields of similar macrocycles using milder conditions [A. D. Sherry et al., *J. Org. Chem*. 54, 2990-2992 (1989)]. Detosylation of the intermediate macrocycle [(3) to yield (4)] was accomplished under acidic conditions in good yield. Reductive detosylation procedures are also well known in the literature and can be adapted to the present reaction sequence. Phosphonomethylation to obtain the tris-aminophosphonic acid derivative (5, PCTMP) was conducted under typical Mannich base conditions using phosphorous acid and formaldehyde.

In addition to phosphonic acid derivatives, phosphonate esters [e.g. of formula (6)] can also be prepared under organic conditions in alcohols or aprotic solvents (e.g. acetonitrile, benzene, toluene, tetrahydrofuran) and using the desired dialkylphosphite as the nucleophilic species (see Scheme 2). Depending upon the reactivity of the amine, these reactions may be conducted at a temperature between -10 to 100°C. In addition, trialkylphosphites can be employed under similar Mannich conditions to give the phosphonate ester via oxidation of phosphorous (III) to phosphorous (V) with simultaneous expulsion of one mole of alcohol (Arbuzov reaction). These reactions can be conducted with or without the presence of a solvent. When alcohols are employed as the solvent for either dialkyl or trialkyl phosphite reactions, it is beneficial to use the alcohol from which the corresponding phosphonate ester is derived in order to avoid alternative products arising from transesterification. Esters of this type are also prepared via N-alkylation of 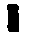 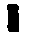 -halodialkylphosphonates in solvents such as acetonitrile, chloroform, dimethylformamide, tetrahydrofuran or 1,4-dioxane with or without the addition of a non-nucleophilic base such as potassium carbonate at room temperature or above. The resulting perester intermediate is then readily hydrolyzed under basic conditions (aqueous hydroxide, pH = 8-14, 30-110°C) to give the corresponding half-acid derivative.

In Scheme 3, macrocyclic methylphosphinic acids (10 and 11) are prepared under conditions similar to those described in Scheme 2. Using diethoxymethylphosphine as the nucleophilic species and paraformaldehyde, condensation can be conducted in solvents such as tetrahydrofuran, dimethylformamide, dioxane, acetonitrile or alcoholic media. The resulting phosphinate ester is then hydrolyzed under acid (6N HCl, 80-100°C) or basic (stoichiometric quantities of base, 40-100°C) conditions to give the corresponding methylphosphonic acid. Alternatively, the method devised by A. D. Sherry et al. (*Inorg. Chem.*, submitted 1991) using ethylphosphonic acid generated *in situ* can be used to obtain phosphinate derivatives having increased lipophilic character.

Scheme 4 illustrates an approach to incorporate additional functionality into the pyridine unit of the 12-membered tertaazamacrocycle. Thus, chelidamic acid (Sigma Chemical Company; 12) can be converted to the bis-halomethyl derivative (13) having appropriate substitution at the pyridyl 4-position. Transformations leading to this intermediate are general in nature and its preparation is described by Takalo et al. [*Acta Chemica Scandinavica* B 42, 373-377 (1988)]. Subsequent macrocyclization using this intermediate (15) can be accomplished by the standard DMF reaction at 100°C with the sodiotritosylated triamine, or at room temperature with the tritosylated free base and potassium carbonate, sodium carbonate, or cesium carbonate as base to give products similar to those previously described. Subsequent reactions leading to phosphonate half-acids and phosphinate functionality are identical to those transformations and conditions described in the preceding Schemes.

In Scheme 4, 4-halopyridyl substituted macrocycles (16) are described which can undergo substitution at the 4-position of the pyridyl moiety as described in Scheme 5. Thus, organometallic Pd(II) complexes can be employed to facilitate the coupling reaction between phenylacetylene and phenylacetylene derivatives and the pyridyl macrocycle. Typical reaction conditions for this transformation utilize anhydrous conditions with triethylamine as solvent and at reaction temperature between 10 to 30°C for optimum yields. The identical product can also be obtained using Cu(I) phenylacetylide in anhydrous pyridine at a temperature between 80 to 110°C. In addition, standard anionic alkylation procedures can be employed to affect substitution on the pyridine nucleus with, for example, sodioalkoxides in DMF or dioxane at from 80 to 100°C using bases such as potassium carbonate or sodium hydroxide. Macrocyclic tetraazamacrocycles (24, 25, 26, 27, 28) derivatized in this manner are compatible with transformations described in previous Schemes resulting in analogous phosphonate chelants.

A variation of 4-pyridyl substitution is described in Scheme 6 whereby the 4-hydroxypyridyl moiety (29) is alkylated with a bromoalkylnitrile yielding an intermediate ether linked nitrile (31) which is subsequently incorporated into the macrocyclic structure. This type of alkylation procedure is best accomplished under anhydrous conditions in an aprotic solvent such as tetrahydrofuran (THF) and using a non-nucleophilic base such as sodium hydride or butyllithium at temperatures between from -30 to 80°C. The generality of this approach has been described by Chaubet et al., for acyclic analogs [*Tetrahedron Letters* 31 (40), 5729-5732 (1990)]. The macrocyclic nitrile prepared in this manner can be reduced to the primary amine (36) by standard procedures followed by protection of the primary amine with 2-(t-butoxycarbonyloxyimino)-2-phenylacetonitrile (BOC-ON; 37). Subsequent functionalization of the macrocyclic secondary amines (38, 39, 40, 41, 42, 43) can then be accomplished by the procedures discussed with the additional requirement that the BOC protecting group be removed using trifluoroacetic acid as described in Scheme 6.

Functionalization can also be carried out on the 3-position of the pyridine ring within the macrocyclic structure as illustrated in Scheme 7. Newkome et al. [*Tetrahedron* 39(12), 2001-2008 (1983)] has previously described the synthesis of ethyl 2,6-halomethylnicotinate (45) which serves as the initial starting material in this synthetic route. Thus, the tris-tosylated macrocycle intermediate (46) can be detosylated under acidic conditions (HBr/AcOH, 25-115°C) with simultaneous hydrolysis to yield the nicotinic acid derivative (48), or reduction of the ester in refluxing ethanol prior to detosylation will result in the 3-hydroxymethyl intermediate (47). The nicotinic acid macrocycle can then be substituted into the general scheme for secondary amine functionalization to yield the various types of phosphonate chelants of Formula (I) (49, 50, 51, 52, 53).

In contrast, the 3-hydroxymethyl analog is advantageously protected prior to functionalization of the macrocyclic amines. The benzyl (Bz) protecting group is shown in Scheme 8 since it must be resistant to the severe acid conditions encountered in the detosylation step. After appropriate functionalization of the secondary amines has been accomplished as described in previous Schemes, the benzyl group is removed under mild catalytic hydrogenation conditions (58).

Macrocyclic derivatives can also be prepared as in Schemes 12-14 where both carboxylate and phosphonate cheating functionalities are present in the same molecule. Thus, varying degrees of carboxylate functionality can be introduced under typical aqueous alkylation procedures using bromoacetic acid. Following this step, the remaining amines can be phosphonomethylated by procedures discussed in previous Schemes using formaldehyde and phosphorous acid, dialkyl phosphonates or trialkyl phosphites.

Schemes 15 and 16 delineate a synthetic approach which introduces an aromatic nitrobenzyl substituent at one of the macrocyclic nitrogen positions. Typically, the macrocyclic amine is mono-N-functionalized in an organic solvent such as acetonitrile or DMF at room temperature using a non-nucleophilic base such as potassium carbonate. Additional functionalization of the remaining nitrogen positions is then performed by methods and conditions described in previous Schemes. After the introduction of the desired cheating moieties, the nitro group is reduced using platinum oxide and hydrogen in water. In this form, the cheating agent is compatible with conjugation techniques which will enable attachment to larger synthetic or natural molecules.

Scheme 17 illustrates the synthesis of the macrocyclic compounds (4) where the amines at positions 3 and 9 are reacted with at least two moles of the sodium salt of hydroxymethanesulfonic acid in water at a pH of about 9 to provide the corresponding macrocyclic compound where positions 3 and 9 are the sodium salt of methanesulfonic acid (119). The sulfonic acid group is then displaced using sodium cyanide to form the corresponding cyanomethane derivative (120). The cyano group is hydrolyzed to the carboxylic acid either: simultaneously with the addition of phosphorous acid and formaldehyde; or by sequential reaction with a derivative of phosphorous acid and formaldehyde to form the phosphonic acid at the 6 position (121), followed by acid hydrolysis, at an elevated temperature, of the cyanato groups and any derivative moiety of the phosphorous acid present. The resulting compound is a macrocycle with two carboxylic acid groups at positions 3 and 9 and a phosphonic acid group at position 6. The phosphonomethylation can also be preformed by the methods discussed above.

The metal ions used to form the complexes of this invention are Gd⁺³, Mn⁺², Fe⁺³ and available commercially, e.g. from Aldrich Chemical Company. The anion present is halide, preferably chloride, or salt free (metal oxide).

A "paramagnetic nuclide" of this invention means a metal ion which displays spin angular momentum and/or orbital angular momentum. The two types of momentum combine to give the observed paramagnetic moment in a manner that depends largely on the atoms bearing the unpaired electron and, to a lesser extent, upon the environment of such atoms. The paramagnetic nuclides found to be useful in the practice of the invention are gadolinium (Gd⁺³), iron (Fe⁺³) and manganese (Mn⁺²), with Gd⁺³ being preferred.

The complexes are prepared by methods well known in the art. Thus, for example, see Chelating Agents and Metal Chelates, Dwyer & Mellor, Academic Press (1964), Chapter 7. See also methods for making amino acids in Synthetic Production and Utilization of Amino Acids, (edited by Kameko, et al.) John Wiley & Sons (1974). An example of the preparation of a complex involves reacting a bicyclopolyazamacrocyclophosphonic acid with the metal ion under aqueous conditions at a pH from 5 to 7. The complex formed is by a chemical bond and results in a stable paramagnetic nuclide composition, e.g. stable to the disassociation of the paramagnetic nuclide from the ligand.

The complexes of the present invention are administered at a ligand to metal molar ratio of at least 1:1, preferably from 1:1 to 3:1, more preferably from 1:1 to 1.5:1. A large excess of ligand is undesirable since uncomplexed ligand may be toxic to the animal or may result in cardiac arrest or hypocalcemic convulsions.

The antibodies or antibody fragments which may be used in the conjugates described herein can be prepared by techniques well known in the art. Highly specific monoclonal antibodies can be produced by hybridization techniques well known in the art, see for example, Kohler and Milstein [*Nature*, 256, 495-497 (1975); and *Eur. J. Immunol.*, 6, 511-519 (1976)]. Such antibodies normally have a highly specific reactivity. In the antibody targeted conjugates, antibodies directed against any desired antigen or hapten may be used. Preferably the antibodies which are used in the conjugates are monoclonal antibodies, or fragments thereof having high specificity for a desired epitope(s). Antibodies used in the present invention may be directed against, for example, tumors, bacteria, fungi, viruses, parasites, mycoplasma, differentiation and other cell membrane antigens, pathogen surface antigens, toxins, enzymes, allergens, drugs and any biologically active molecules. Some examples of antibodies or antibody fragrents are 1116-NS-19-9, 1116-NS-3d, 703D4, 704A1, CC49, CC83 and B72.3. All of these antibodies have been deposited in ATCC. A more complete list of antigens can be found in US-A-4,193,983. The conjugates of the present invention are particularly preferred for the diagnosis of various cancers.

This invention is used with a physiologically acceptable carrier, excipient or vehicle therefore. The methods for preparing such formulations are well known. The formulations may be in the form of a suspension, injectable solution or other suitable formulations. Physiologically acceptable suspending media, with or without adjuvants, may be used.

An "effective amount" of the formulation is used for diagnosis. The dose will vary depending on the disease and physical parameters of the animal, such as weight. *In vivo* diagnostics are also contemplated using formulations of this invention.

Other uses of some of the chelants of the present invention may include the removal of undesirable metals (i.e. iron) from the body, attachment to polymeric supports for various purposes, e.g. as diagnostic agents, and removal of metal ions by selective extraction. The ligands of Formula (I) having in at least two R terms T equal to P(O)R¹OH may be used for metal ion control as scale inhibitors. Some of these ligands can be used in less than stoichiometric amounts. Similar uses are known for compounds described in US-A-2,609,390; US-A-3,331,773; US-A-3,336,221; and US-A-3,434,969.

## Claims

**1.** Bicyclopolyazamacrocyclophosphonic acid compounds of the formula 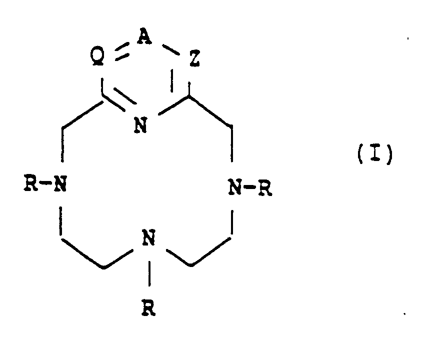 wherein: 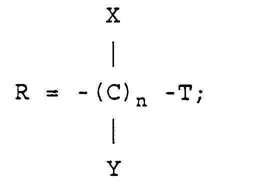 where:
X and Y are independently H, OH, C₁-C₃ alkyl or COOH;
n is an integer of 1, 2 or 3;
with the proviso that: when n is 2, then the sum of X and Y must equal two or more H; and when n is 3, then the sum of X and Y must equal three or more H;
T is H, C₁-C₁₈ alkyl, COOH, OH, SO₃H, 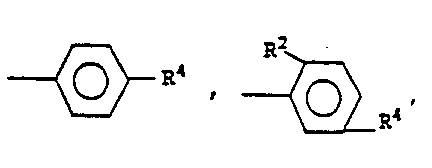 P(O)(OC₁-C₅ alkyl)₂ or P(O)R¹OH:
where:
R¹ is OH, C₁-C₅ alkyl or -O-(C₁-C₅ alkyl);
R² is H or OH; with the proviso that when R² is OH, then the R term containing the R² must have all X and Y equal to H;
R⁴ is H, NO₂, NH₂, isothiocyanato, semicarbazido, thiosemicarbazido, maleimido, bromoacetamido or carboxyl;
with the proviso that, except when any T is P(O)(OC₁-C₅ alkyl)₂, at least one T must be P(O)(OH)₂ and with the proviso that when one T is 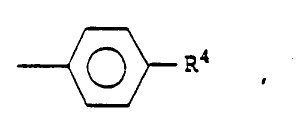 then one X or Y of that R term may be COOH and all other X and Y terms of that R term must be H;
A is CH, N, C-Br, C-Cl, C-OR³, C-OR⁸, N⁺-R⁵X⁻, 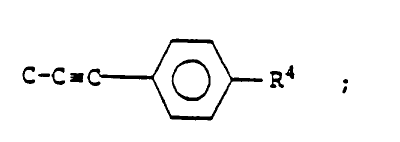
R³ is H, C₁-C₅ alkyl, benzyl, or benzyl substituted with at least one R⁴;
R⁵ is C₁-C₁₆ alkyl, benzyl, or benzyl substituted with at least one R⁴;
R⁸ is C₁-C₁₆ alkylamino;
X⁻ is Cl⁻, Br⁻, I⁻ or H₃CCO₂⁻;
Q and Z independently are CH, N, N⁺-R⁵X⁻, C-CH₂-OR³ or C-C(O)-R⁶,
R⁶ is -O-(C₁-C₃ alkyl), OH or NHR⁷;
R⁷ is C₁-C₅ alkyl or a biologically active material; or pharmaceutically-acceptable salts thereof;
with the proviso that:
a) when Q, A or Z is N or N⁺ -R⁵X⁻, then the other two groups must be CH;
b) when A is C-Br, C-Cl, C-OR³ or C-OR⁸, then both Q and Z must be CH;
c) the sum of the R⁴, R⁷ and R⁸ terms, when present, may not exceed one;
d) only one of Q or Z cab be C-C(O)-R⁶ and when one of Q or Z is C-C(O)-R⁶, then A must be CH;
e) when all R¹ groups are OH and no T group contains an R⁴ group, at least one of A, Q and Z is not CH or N; and
f) when T in one R term is P(O)(OC₁-C₅ alkyl)₂, each T in the remaining two R terms independently is P(O)(OC₁-C₅ alkyl)₂ or P(O)(OH)(OC₁-C₅ alkyl).

**2.** A compound of Claim 1 wherein X and Y are H.

**3.** A compound of Claim 1 or Claim 2 wherein n is 1.

**4.** A compound of any one of Claims 1 to 3 wherein A, Q and Z are CH.

**5.** A compound of any one of Claims 1 to 4 wherein two R terms have T equal to P(O)R¹OH, where R¹ is OH and the third T equals H, COOH or C₁-C₁₈ alkyl; A, Q and Z are CH; n is 1, and X and Y independently are H or C₁-C₃ alkyl.

**6.** A compound of Claim 5 wherein in two R terms T is P(O)R¹OH, where R¹ is OH, and in the third R term T is COOH, and n is 1.

**7.** A compound of any one of Claims 1 to 4 wherein at least one R term has T equal to P(O)R¹OH, where R¹ is -O-(C₁-C₅ alkyl).

**8.** A compound of any one of Claims 1 to 4 wherein:
(a) three R terms have T equal to P(O)R¹OH where R¹ is OH; and X and Y are H, and named as 3,6,9,15-tetraazabicyclo[9.3.1]-pentadeca-1(15),11,13-triene-3,6,9-trimethylenephosphonic acid or pharmaceutically-acceptable salt thereof;
(b) in two R terms T is P(O)R¹OH, where R¹ is OH, in the third R term T is P(O)R¹OH, where R¹ is C₁-C₅ alkyl, and n is 1; or
(c) in two R terms T is P(O)R¹OH, where R¹ is OH, in the third R term T is P(O)R¹OH, where R¹ is -O-(C₁-C₅ alkyl), and n is 1.
Y, A, Q and Z are defined as in Claim 1.

**10.** A compound of Claim 9 wherein:
(a) in one R term T is P(O)R¹OH, where R¹ is OH, and in the other two R terms T is P(O)R¹OH, where R¹ is -O-(C₁-C₅ alkyl), and n is 1;
(b) in one R term T is P(O)R¹OH, where R¹ is OH, and in the other two R terms T is P(O)R¹OH, where R¹ is C₁-C₅ alkyl, and n is 1; or
(c) in one R term T is P(O)R¹OH, where R¹ is OH, and in the other two R terms T is COOH, and n is 1.

**11.** A compound of any one of Claims 1 to 4 wherein n is 1, X and Y are H; T is COOH or 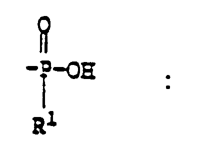 where: R¹ is -OH, C₁-C₅ alkyl or -O-(C₁-C₅ alkyl).

**12.** A compound of Claim 11 wherein:
(a) Q and Z are CH, A is C-OR³, C-OR⁸, where R³ and R⁸ are defined as in Claim 1, or 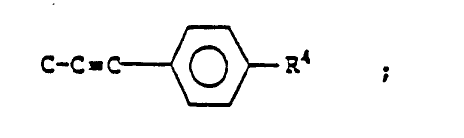 where R⁴ is defined as in Claim 1 or where R⁴ is defined as in Claim 1 or
(b) A is CH, and one of Q or Z is CH and the other is C-C(O)-R⁶, where R⁶ is defined as in Claim 1.

**13.** A compound of type (b) Claim 12 wherein R⁶ is NHR⁷, where R⁷ is a biologically active material.

**14.** A complex which comprises a bicyclopolyazamacrocyclophosphonic acid compound as claimed in any one of Claims 1 to 13 complexed with a metal ion selected from Gd⁺³, Mn⁺² or Fe⁺³.

**15.** A complex of Claim 14 wherein the metal is Gd⁺³.

**16.** A complex of Claim 14 having an overall charge of +1.

**17.** A conjugate comprising a bicyclopolyazamacrocyclophosphonic acid complex as claimed in Claim 14 or Claim 15, with the proviso that one of R⁴, R⁷ or R⁸ must be present, covalently attached to a biologically active material.

**18.** A conjugate of Claim 17 wherein the biologically active material is a dextran, a polypeptide, a molecule that has specific affinity for a receptor, an antibody or antibody fragment.

**19.** A conjugate of Claim 17 wherein the antibody or antibody fragment is a monoclonal antibody or fragment thereof.

**20.** A conjugate of any one of Claims 17 to 19 wherein A is CH, and one of Q or Z is CH and the other is C-C(O)-R⁶, where R⁶ is NHR⁷, where R⁷ is a biologically active material.

**21.** A conjugate of any one of Claims 17 to 19 wherein three R terms have T equal to P(O)R¹OH where R¹ is OH; and X and Y are H or pharmaceutically-acceptable salts thereof.

**22.** A pharmaceutical formulation comprising a complex of Claims 14 or Claim 16 with a pharmaceutically-acceptable carrier.

**23.** A pharmaceutical formulation comprising a conjugate of any one of Claims 17 to 21 with a pharmaceutically-acceptable carrier.

**24.** The complex as claimed in any one of Claims 14 to 16 for use as a pharmaceutical.

**25.** The conjugate as claimed in any one of Claims 17 to 21 for use as a pharmaceutical.

**26.** A kit for use as a diagnostic agent having as an ingredient a ligand as claimed in any one of Claims 1 to 13.

**27.** A process for preparing a complex as claimed in Claim 14 which comprises reacting a bicyclopolyazamacrocyclophosphonic acid compound as claimed in Claim 1 with a metal ion selected from Gd⁺³, Mn⁺² or Fe⁺³ under aqueous conditions at a pH from 5 to 7.

**28.** A process for preparing a bicyclopolyazamacrocyclophosphonic acid compound as claimed in Claim 1 which comprises reacting:
(A) a compound of the Formula (I) wherein at least 1 R group is H, with a phosphonating agent; or
(B) a compound of Formula (I) wherein Q, A or Z has a protecting group present, after step (A), removing the blocking group by catalytic hydrogenation or acid.

**29.** The process of Claim 28 wherein the phosphonating agent has the formula P(OR)₃ where R is defined as in Claim 1.

**30.** The process of Claim 28 wherein the phosphonating agent has the formula P(OR)₃ where R is defined as in Claim 1, and formaldehyde is a solvent.
